(19) Europäisches Patentamt
European Patent Office
Office européen des brevets

(11) EP 4 640 148 A1

(12) **EUROPÄISCHE PATENTANMELDUNG**

(43) Veröffentlichungstag:
29.10.2025 Patentblatt 2025/44

(21) Anmeldenummer: 25168722.4

(22) Anmeldetag: 07.04.2025

(51) Internationale Patentklassifikation (IPC):
*A61B 5/08* (2006.01)    *A61B 5/00* (2006.01)

(52) Gemeinsame Patentklassifikation (CPC):
**A61B 5/0816; A61B 5/7203; A61B 5/7278;
A61B 5/7289;** A61B 5/024; A61B 5/7246

(84) Benannte Vertragsstaaten:
**AL AT BE BG CH CY CZ DE DK EE ES FI FR GB
GR HR HU IE IS IT LI LT LU LV MC ME MK MT NL
NO PL PT RO RS SE SI SK SM TR**
Benannte Erstreckungsstaaten:
**BA**
Benannte Validierungsstaaten:
**GE KH LA MA MD TN**

(30) Priorität: **19.04.2024 DE 102024111079**

(71) Anmelder: **Drägerwerk AG & Co. KGaA
23558 Lübeck (DE)**

(72) Erfinder: **KAHL, Lorenz
23558 Lübeck (DE)**

(54) **SIGNALVERARBEITUNGSEINHEIT UND VERFAHREN ZUR ERMITTLUNG EINES KARDIOGENEN SIGNALS**

(57) Die Erfindung betrifft eine Signalverarbeitungseinheit und ein Verfahren, welche eine Schätzung ($Sig_{res,est}$) für ein respiratorisches Signal ermitteln. Dieses respiratorische Signal ist ein Maß für die eigene spontane Atmungsaktivität und / oder die künstliche Beatmung eines Patienten. Aus Messwerten, die im / oder am Körper des Patienten gemessen werden, wird ein Summen-Signal ($Sig_{Sum}$) erzeugt, welches das respiratorische und ein kardiogenes Signal umfasst. Indem der Einfluss der Herzaktivität auf das Summen-Signal ($Sig_{Sum}$) kompensiert wird, wird ein Zwischen-Signal ($Sig_{com}$) berechnet. Ein Referenz-Dämpfungs-Signalab-schnitt beschreibt den durchschnittlichen zeitlichen Verlauf des Beitrags des kardiogenen Signals zum Zwischen-Signal ($Sig_{com}$). Für jeden detektierten Herzschlag wird ein Zwischen-Signalabschnitt [$SigA_{com}(x)$] erzeugt. Aus diesem wird ein gedämpfter Zwischen-Signalabschnitt [$SigA_{com,d}(X)$] erzeugt, wofür ein angepasster Dämpfungs-Signalabschnitt [Mod(1)(x), ..., Mod(n)(x)] auf den Zwischen-Signalabschnitt angewendet wird. Der angepasste Dämpfungs-Signalabschnitt [Mod(1)(x), ..., Mod(n)(x)] wird aus dem Referenz-Dämpfungs-Signalabschnitt unter Verwendung mindestens eines Qualitätsmaßes [Q(30), Q(31)] berechnet.

FIG. 14

## Beschreibung

Die Erfindung betrifft eine Signalverarbeitungseinheit und ein Verfahren, welche eine Schätzung für ein respiratorisches Signal ermitteln. Dieses respiratorische Signal ist ein Maß für die eigene Atmungsaktivität und / oder eine künstliche Beatmung eines Patienten. Die eigene Atmungsaktivität des Patienten wird durch seine spontane Atmung und / oder durch eine externe Stimulation seiner Atmungsmuskulatur bewirkt. Sowohl die eigene Atmungsaktivität als auch die künstliche Beatmung bewirken eine Ventilation der Lunge des Patienten. Das respiratorische Signal wird beispielsweise dafür benötigt, um den Zustand der Atmungsmuskulatur des Patienten zu ermitteln oder um die künstliche Beatmung an die eigene Atmungsaktivität des Patienten anzupassen.

In der Regel lässt sich das respiratorische Signal nicht direkt messen. Vielmehr lässt sich nur ein Signal messen, welches aus einer Überlagerung des gesuchten respiratorischen Signals mit einem kardiogenen Signal und optional mit Störsignalen resultiert und Summen-Signal genannt wird. Das kardiogene Signal ist ein Maß für die Herzaktivität des Patienten.

Der Erfindung liegt die Aufgabe zugrunde, eine Signalverarbeitungseinheit und ein Verfahren bereitzustellen, welche besser als bekannte Signalverarbeitungseinheiten und Verfahren aus einem Summen-Signal ein respiratorisches Signal zu gewinnen vermögen, wobei das Summen-Signal aus Messwerten, die an einem Patienten gemessen worden sind, erzeugt worden ist und eine Überlagerung des respiratorischen und eines kardiogenen Signals umfasst und wobei bei den Messungen Störungen auftreten können. Anders formuliert: das respiratorische Signal soll mit höherer Zuverlässigkeit aus dem Summensignal gewonnen werden können.

Die Aufgabe wird durch eine Signalverarbeitungseinheit mit den Merkmalen des Anspruchs 1 und durch ein Verfahren mit den Merkmalen des Anspruchs 7 gelöst. Vorteilhafte Ausgestaltungen der erfindungsgemäßen Signalverarbeitungseinheit sind, soweit sinnvoll, auch vorteilhafte Ausgestaltungen des erfindungsgemäßen Verfahrens und umgekehrt.

Die erfindungsgemäße Signalverarbeitungseinheit und das erfindungsgemäße Verfahren vermögen eine Schätzung für ein respiratorisches Signal zu ermitteln. Das zu schätzende respiratorische Signal korreliert mit der Ventilation der Lunge eines Patienten, also mit der Belüftung und Entlüftung der Lunge. Die Ventilation der Lunge wird durch die eigene Atmungsaktivität und / oder durch eine künstliche Beatmung des Patienten erzeugt. Die eigene Atmungsaktivität wird von der Atmungsmuskulatur des Patienten erzeugt, in der Regel durch seine spontane Atmung und in einer Ausgestaltung zusätzlich oder stattdessen durch eine externe Stimulation der Atmungsmuskulatur, z. B. durch die künstliche Beatmung oder in einem Magnetfeld.

In einer rechnerauswertbaren Form sind ein Referenz-Herzschlag-Zeitraum und eine Nutzphase vorgegeben. Der Referenz-Herzschlag-Zeitraum lässt sich dafür verwenden, um den typischen Verlauf eines Herzschlags zu beschreiben - genauer gesagt: eines kardiogenen Signals im Verlaufe eines Herzschlag-Zeitraums. In der Nutzphase wird die Schätzung des respiratorischen Signals ermittelt.

Mindestens ein Summen-Signal-Sensor vermag ein Signal zu messen, welches im und / oder am Körper des Patienten erzeugt wird, beispielsweise mithilfe von Messelektroden oder mit Hilfe eines Messinstruments im Körper des Patienten. Optional werden mehrere Summen-Signal-Sensoren verwendet. Der oder ein Summen-Signal-Sensor umfasst in einer Ausgestaltung mehrere Elektroden, die auf der Haut des Patienten positioniert sind. Das am oder im Körper des Patienten erzeugte Signal wird von der eigenen Atmungsaktivität und / oder der künstlichen Beatmung des Patienten sowie von seiner Herzaktivität generiert.

Erfindungsgemäß erzeugt die Signalverarbeitungseinheit mindestens ein Summen-Signal und verwendet (verarbeitet) hierfür Messwerte des oder mindestens eines Summen-Signal-Sensors. Optional erzeugt die Signalverarbeitungseinheit für jeden Summen-Signal-Sensor jeweils ein Summen-Signal. Das oder jedes erzeugte Summen-Signal umfasst eine Überlagerung des respiratorischen Signals, das näherungsweise ermittelt werden soll, und eines kardiogenen Signals. Das kardiogenen Signal beschreibt die Herzaktivität des Patienten. Optional fließt in das Summen-Signal mindestens ein Störsignal ein.

Die Signalverarbeitungseinheit detektiert unter Verwendung des oder mindestens eines Summen-Signals mehrere Herzschläge, welche der Patient in der Nutzphase durchführt. Weiterhin detektiert die Signalverarbeitungseinheit für jeden detektierten Herzschlag jeweils einen charakteristischen Herzschlag-Zeitraum. Der Herzschlag findet in diesem Herzschlag-Zeitraum statt. In diesem Herzschlag-Zeitraum wird das Summen-Signal im Wesentlichen vom kardiogenen Signal festgelegt, und zwar über den gesamten Herzschlag-Zeitraum oder wenigstens in Teil-Zeiträumen, und außerhalb eines Herzschlag-Zeitraums im Wesentlichen vom respiratorischen Signal. Das entsprechende gilt auch für den Referenz-Herzschlag-Zeitraum.

Die Signalverarbeitungseinheit erzeugt ein Zwischen-Signal. Um das Zwischen-Signal zu erzeugen, kompensiert die Signalverarbeitungseinheit wenigstens näherungsweise rechnerisch den Einfluss der Herzaktivität, also des kardiogenen Signals, auf das Summen-Signal, beispielsweise durch Subtraktion. Bevorzugt ist das Zwischen-Signal das Ergebnis dieser Kompensation.

Die Signalverarbeitungseinheit ermittelt mindestens einen Referenz-Dämpfungs-Signalabschnitt. In einer ersten Alternative berechnet die Signalverarbeitungseinheit den Referenz-Dämpfungs-Signalabschnitt, in einer zweiten Alternative ermittelt sie ihn durch einen Lesezugriff auf einen Datenspeicher. Der oder jeder ermittelte Referenz-Dämpfungs-Signalabschnitt korreliert mit dem durchschnittlichen zeitlichen Verlauf des Beitrags des kardiogenen Signals zum Zwischen-Signal, und zwar mit dem Beitrag im vorgegebenen Referenz-Herzschlag-Zeitraum. Der Referenz-Dämpfungs-Signalabschnitt bezieht sich auf den Referenz-Herzschlag-Zeitraum und gilt wenigstens näherungsweise für eine Vielzahl von Herzschlag-Zeiträumen dieses Patienten.

Das zu ermittelnde respiratorische Signal bezieht sich auf die Nutzphase. Für jeden detektierten Herzschlag, der in die Nutzphase fällt, werden die folgenden Schritte durchgeführt:

- Wie bereits erwähnt, wird jeweils ein charakteristischer Herzschlag-Zeitraum für den detektierten Herzschlag detektiert. Die Signalverarbeitungseinheit erzeugt einen Zwischen-Signalabschnitt als einen Abschnitt des Zwischen-Signals. Der Zwischen-Signalabschnitt liegt im Herzschlag-Zeitraum dieses Herzschlags und ist beispielsweise derjenige Abschnitt des Zwischen-Signals, der in den Herzschlag-Zeitraum fällt. Für jeden detektierten Herzschlag wird also jeweils ein Zwischen-Signalabschnitt erzeugt.
- Die Signalverarbeitungseinheit erzeugt für jeden detektierten Herzschlag jeweils einen gedämpften Zwischen-Signalabschnitt.
- Um den gedämpften Zwischen-Signalabschnitt zu erzeugen, wendet die Signalverarbeitungseinheit bei der ersten Alternative den Referenz-Dämpfungs-Signalabschnitt und bei der zweiten Alternative einen angepassten Dämpfungs-Signalabschnitt auf den Zwischen-Signalabschnitt an.

Der gedämpfte Zwischen-Signalabschnitt für einen detektierten Herzschlag hat folgende Eigenschaft: Der Einfluss des kardiogenen Signals auf den gedämpften Zwischen-Signalabschnitt ist kleiner als oder höchstens genauso groß wie der Einfluss des kardiogenen Signals auf den (nicht gedämpften) Zwischen-Signalabschnitt, aber nicht größer. Idealerweise nimmt das kardiogene Signal überhaupt keinen Einfluss auf den gedämpften Zwischen-Signalabschnitt.

Die Signalverarbeitungseinheit setzt die gedämpften Zwischen-Signalabschnitte zur gesuchten Schätzung für das respiratorische Signal zusammen. Für dieses Zusammensetzen verwendet die Signalverarbeitungseinheit die detektierten charakteristischen Herzschlag-Zeitpunkte. Dadurch werden die gedämpften Zwischen-Signalabschnitte zeitrichtig zusammengesetzt. Optional werden Zwischenräume zwischen benachbarten gedämpften Zwischen-Signalabschnitten unter Verwendung von entsprechenden Abschnitten des Zwischen-Signals miteinander verbunden.

Die Signalverarbeitungseinheit berechnet mindestens eines von vier Qualitätsmaßen. Drei der vier Qualitätsmaße beschreiben die jeweilige Zuverlässigkeit der folgenden Ermittlungen und Berechnungen:

- die Zuverlässigkeit, mit der der oder jeder verwendete Summen-Signal-Sensor die jeweiligen Messwerte misst und / oder die Zuverlässigkeit, mit der die Signalverarbeitungseinheit aus diesen Messwerten das jeweilige Summen-Signal erzeugt,
- für mindestens einen Herzschlag, bevorzugt für mehrere Herzschläge, jeweils die Zuverlässigkeit, mit der die Signalverarbeitungseinheit in der Nutzphase den jeweiligen charakteristischen Herzschlag-Zeitpunkt dieses Herzschlags detektiert hat,
- die Zuverlässigkeit, mit der ein Referenz-Dämpfungs-Signalabschnitt ermöglicht, den Beitrag des kardiogenen Signals zum Zwischen-Signal im Referenz-Herzschlag-Zeitraum rechnerisch zu kompensieren.

Das vierte Qualitätsmaß bewertet die Form des jeweiligen Zwischen-Signalabschnitts für einen Herzschlag.

Bevorzugt ist das Qualitätsmaß umso größer, je besser die jeweilige Bewertung ist. Falls das Qualitätsmaß umso kleiner ist, je besser die Bewertung ist, so ist die nachfolgende Beschreibung entsprechend abzuändern.

Gemäß der ersten Alternative wendet die Signalverarbeitungseinheit den Referenz-Dämpfungs-Signalabschnitt auf den Zwischen-Signalabschnitt an. Diesen Referenz-Dämpfungs-Signalabschnitt hat die Signalverarbeitungseinheit zuvor unter Verwendung mindestens eines der oben genannten vier Qualitätsmaße berechnet, und zwar bevorzugt mit Hilfe einer Stichprobe und bevorzugt vor der Nutzungsphase. Möglich ist, dass die Signalverarbeitungseinheit während der Nutzungsphase den Referenz-Dämpfungs-Signalabschnitt laufend aktualisiert.

Gemäß der zweiten Alternative wendet die Signalverarbeitungseinheit einen angepassten Dämpfungs-Signalabschnitt auf den Zwischen-Signalabschnitt an. Der angepasste Dämpfungs-Signalabschnitt für einen detektierten Herzschlag wird durch die folgenden Schritte erzeugt:
Die Signalverarbeitungseinheit berechnet den angepassten Dämpfungs-Signalabschnitt, wofür sie den ermittelten, also berechneten oder durch Lesezugriff ermittelten, Referenz-Dämpfungs-Signalabschnitt und mindestens ein berechnetes Qualitätsmaß verwendet.

Die Berechnung geschieht wie folgt:

- Der angepasste Dämpfungs-Signalabschnitt ist kleiner als oder höchstens genauso groß wie der Referenz-Dämpfungs-Signalabschnitt.

- Der angepasste Dämpfungs-Signalabschnitt ist umso kleiner, je kleiner das oder jedes berechnete und angewendete Qualitätsmaß ist.

Das tatsächliche kardiogene Signal wird mit $Sig_{kar}$ bezeichnet, das tatsächliche respiratorische Signal mit $Sig_{res}$. Die erfindungsgemäß erzeugte Schätzung für das respiratorische Signal $Sig_{res}$ wird mit $Sig_{res,est}$ bezeichnet. In vielen Fällen lassen sich von Messwerten von optionalen weiteren Sensoren ein Maß $P_{aw}$ (pressure in airway) für den Atemwegsdruck und / oder ein Maß $P_{es}$ (pressure in esophagus) für den Speiseröhrendruck herleiten. Aus diesen Maßen lässt sich ein pneumatisches Maß $P_{mus}$ ableiten, welches ebenfalls ein Maß für die eigene Atmungsaktivität des Patienten ist. Indem einerseits eine Schätzung $Sig_{res,est}$ für das elektrische oder mechanische respiratorische Signal $Sig_{res}$ und andererseits ein pneumatisches Maß $P_{mus}$ ermittelt werden, wird die eigene Atmungsaktivität des Patienten mit höherer Zuverlässigkeit als bei der Herleitung nur eines Signals ermittelt, und es lässt sich ableiten, wie gut die Atmungsmuskulatur des Patienten elektrische Reize im Körper des Patienten in pneumatische Atmungsaktivität umsetzt (neuromechanische Effizienz). Die Erfindung lässt sich auch in einer Ausgestaltung verwenden, in welcher zwar das EMG-Signal oder das MMG-Signal erzeugt wird, aber nicht das pneumatische Maß $P_{mus}$ für die Atmungsaktivität.

Das erfindungsgemäß ermittelte geschätzte respiratorische Signal $Sig_{res,est}$ wird beispielsweise für die folgenden Zwecke verwendet:

- Die neuromechanische Effizienz der Atmungsmuskulatur des Patienten wird ermittelt.
- Der Zustand der Atmungsmuskulatur des Patienten wird ermittelt (insbesondere eine Fatigue-Ermittlung) - hierfür wird das pneumatische Maß $P_{mus}$ nicht benötigt,
- Asynchronien in der eigenen Atmungsaktivität des Patienten werden detektiert - auch hierfür wird das pneumatische Maß $P_{mus}$ nicht benötigt,
- Um den Patienten zu überwachen, werden das geschätzte respiratorische Signal $Sig_{res,est}$ und die respiratorische EMG-Leistung ermittelt und als zwei Vitalparameter in einer von einem Menschen wahrnehmbaren Form ausgegeben, bevorzugt visuell in Form jeweils eines zeitlichen Verlaufs, optional zusammen mit dem Atemwegsdruck $P_{aw}$ und / oder dem Speiseröhrendruck $P_{es}$,
- Der Patient führt eine eigene Atmungsaktivität aus und wird unterstützend künstlich beatmet. Ein pneumatisches Maß $P_{mus}$ für die eigene Atmungsaktivität des Patienten wird unter Verwendung des respiratorischen Signals $Sig_{res,est}$ hergeleitet. Die künstliche Beatmung, die ein Beatmungsgerät bewirkt, wird so gut als möglich mit der eigenen Atmungsaktivität des Patienten synchronisiert. Bei der künstlichen Beatmung führt bevorzugt das Beatmungsgerät eine Folge von Beatmungshüben (ventilation strokes) aus, und in jedem Beatmungshub wird jeweils eine Menge eines Gasgemischs umfassend Sauerstoff zum Patienten gefördert. Bevorzugt werden die Beatmungshübe der künstlichen Beatmung abhängig vom geschätzten respiratorischen Signal $Sig_{res,est}$ durchgeführt. Beispielsweise löst das Beatmungsgerät die Beatmungshübe abhängig vom geschätzten respiratorischen Signal $Sig_{res,est}$ aus und / oder beendet sie und / oder legt die jeweilige Amplitude jedes Beatmungshubs und / oder die zeitlich veränderliche Frequenz der Beatmungshübe abhängig von dem geschätzten respiratorischen Signal $Sig_{res,est}$ fest. Auch das Ende der künstlichen Beatmung kann abhängig von dem geschätzten respiratorischen Signal $Sig_{res,est}$ geregelt werden.

Erfindungsgemäß wird aus dem Summen-Signal ein Zwischen-Signal berechnet. Im Zwischen-Signal ist der Einfluss der Herzaktivität, also des kardiogenen Signals, auf das Summen-Signal näherungsweise rechnerisch kompensiert. Dieses Zwischen-Signal wird erfindungsgemäß gedämpft. Der Erfindung liegt folgende Erkenntnis zugrunde: In einem gesamten Herzschlag-Zeitraum oder wenigstens in einem Abschnitt des Herzschlag-Zeitraums ist der Einfluss des kardiogenen Signals $Sig_{kar}$ auf das Summen-Signal erheblich größer, bevorzugt mindestens 50 mal größer, besonders bevorzugt mindestens 100 mal größer, als der Einfluss des respiratorischen Signals $Sig_{res}$. Hingegen wird das Summen-Signal im Zeitraum zwischen zwei aufeinanderfolgenden Herzschlag-Zeiträumen überwiegend oder sogar ausschließlich vom respiratorischen Signal $Sig_{res}$ bestimmt. Idealerweise ist im Zwischen-Signal der Einfluss der Herzaktivität vollständig kompensiert, in der Praxis nur teilweise. Die Dämpfung kompensiert wenigstens näherungsweise denjenigen Einfluss des kardiogenen Signals $Sig_{kar}$ auf das Zwischen-Signal, der nach der rechnerischen Kompensation noch verbleibt.

Die erfindungsgemäße Verwendung mindestens eines Qualitätsmaßes berücksichtigt insbesondere die Tatsache, dass im Verlaufe der Nutzphase beim Vorgang, dass der oder mindestens ein Summen-Signal-Sensor das jeweilige Summen-Signal misst, verschiedene Störungen auftreten können. Diese Störungen können dazu führen, dass im Zwischen-Signal noch ein relativ großer kardiogener Signalanteil erhalten ist. Dieser Anteil wird abhängig vom generierten Qualitätsmaß reduziert.

Gemäß der ersten Alternative berechnet die Signalverarbeitungseinheit einen Referenz-Dämpfungs-Signalabschnitt. Bevorzugt berechnet die Signalverarbeitungseinheit in einer Initialisierungsphase den Referenz-Dämpfungs-Signalabschnitt, wobei die Initialisierungsphase zeitlich vor der Nutzphase liegt. Bevorzugt werden die Initialisierungsphase und die Nutzphase für denselben Patienten durchgeführt. Für die Berechnung verwendet die Signalverarbeitungseinheit eine Stichprobe mit mehreren Stichprobenelementen. Jedes Stichprobenelement bezieht sich auf jeweils einen Herzschlag. Jedes Stichprobenelement umfasst jeweils einen Zwischen-Signalabschnitt. Dieser Zwischen-Signalabschnitt ist ein Abschnitt des Zwischen-Signals und liegt im Herzschlag-Zeitraum dieses Herzschlags.

Für jedes Stichprobenelement generiert die Signalverarbeitungseinheit jeweils ein Leistungsmaß-Stichprobenelement. Das Leistungsmaß-Stichprobenelement ist der zeitliche Verlauf eines Maßes für die elektrische Leistung im Herzschlag-Zeitraum des Herzschlags.

Die Signalverarbeitungseinheit erzeugt aus den Stichprobenelementen einen durchschnittlichen Leistungs-Signalabschnitt, nämlich als gewichtetes Mittel über die Leistungsmaß-Stichprobenelemente. Das gewichtete Mittel umfasst Gewichtsfaktoren. Diese Gewichtsfaktoren werden unter Verwendung eines Leistungs-Qualitätsmaßes berechnet. Der Gewichtsfaktor für ein Leistungsmaß-Stichprobenelement ist umso kleiner, je kleiner das Leistungs-Qualitätsmaß für diese Stichprobenelement ist. Das Leistungs-Qualitätsmaß ist ein Qualitätsmaß, welches die Form des Leistungs-Signalabschnitts des Stichprobenelements beschreibt. Möglich ist, dass alle Gewichtsfaktoren gleich groß sind, dass also ein arithmetisches Mittel gebildet wird.

Die Signalverarbeitungseinheit verwendet den berechneten durchschnittlichen Leistungssignal-Abschnitt, um den Referenz-Dämpfungs-Signalabschnitt zu berechnen.

In einer Ausgestaltung werden mehrere Frequenzbänder vorgegeben. Mehrere gerade beschriebene Schritte werden für jedes Frequenzband durchgeführt, bevorzugt parallel durchgeführt. Insbesondere führt die Signalverarbeitungseinheit für jedes vorgegebene Frequenzband folgenden Schritt durch: Sie berechnet jeweils einen Anteil des Referenz-Dämpfungs-Signalabschnitts oder ermittelt den Anteil durch eine Lesezugriff auf den Datenspeicher. Jeder Anteil bezieht sich auf jeweils ein Frequenzband.

Für jedes vorgegebene Frequenzband und für jeden im der Nutzphase detektierten Herzschlag führt die Signalverarbeitungseinheit weiterhin folgende Schritte durch:

- Sie erzeugt einen Anteil des Zwischen-Signalabschnitts für den Herzschlag-Zeitraum dieses detektierten Herzschlags, wobei der Anteil in diesem Frequenzband auftritt.
- Sie erzeugt einen Anteil des gedämpften Zwischen-Signalabschnitt für den Herzschlag-Zeitraum, wobei dieser Anteil in dem Frequenzband auftritt. Hierfür verwendet sie den Anteil des Zwischen-Signalabschnitts für dieses Frequenzband.

Die Signalverarbeitungseinheit erzeugt den gedämpften Zwischen-Signalabschnitt für einen Herzschlag-Zeitraum aus den Anteilen für die Frequenzbänder, die so wie gerade beschrieben berechnet wurden.

Bei der ersten Alternative führt die Signalverarbeitungseinheit folgende Schritte durch:

- Wie gerade beschrieben, hat die Signalverarbeitungseinheit für jeden detektierten Herzschlag jeweils einen Anteil des Zwischen-Signalabschnitts für den Herzschlag berechnet. Dieser Anteil bezieht sich auf das Frequenzband.
- Die Signalverarbeitungseinheit ermittelt für jedes Frequenzband jeweils einen Anteil des Referenz-Dämpfungs-Signalabschnitts. Dieser Anteil bezieht sich ebenfalls auf das Frequenzband. Um diesen Anteil zu berechnen, verwendet die Signalverarbeitungseinheit mindestens ein Qualitätsmaß.
- Die Signalverarbeitungseinheit wendet den Anteil des Referenz-Dämpfungs-Signalabschnitts auf den Anteil des Zwischen-Signalabschnitts an. Diese Anwendung liefert einen Anteil des gedämpften Zwischen-Signalabschnitts.

Bei der zweiten Alternative führt die Signalverarbeitungseinheit folgende Schritte durch:

- Die Signalverarbeitungseinheit ermittelt für jedes Frequenzband jeweils einen Anteil des Referenz-Dämpfungs-Signalabschnitts. Dieser Anteil bezieht sich auf das Frequenzband.
- Die Signalverarbeitungseinheit berechnet für jeden detektierten Herzschlag und für jedes Frequenzband jeweils einen Anteil des angepassten Dämpfungs-Signalabschnitts. Dieser Anteil bezieht sich auf das Frequenzband. Um diesen Anteil zu berechnen, verwendet die Signalverarbeitungseinheit wenigstens ein Qualitätsmaß.
- Die Signalverarbeitungseinheit wendet den Anteil des angepassten Dämpfungs-Signalabschnitts auf den Anteil des Zwischen-Signalabschnitts an. Die Anwendung liefert einen Anteil des gedämpften Zwischen-Signalabschnitts.

Die Erfindung betrifft weiterhin eine Anordnung mit einer erfindungsgemäßen Signalverarbeitungseinheit und mindestens einem Summen-Signal-Sensor. Der oder jeder Summen-Signal-Sensor der Anordnung vermag ein Signal zu messen, das im und / oder am Körper des Patienten erzeugt wird. Der oder jeder Summen-Signal-Sensor liefert Messwerte. Die Signalverarbeitungseinheit empfängt diese Messwerte und erzeugt aus empfangenen Messwerten mindestens ein Summen-Signal, in einer Ausgestaltung für jeden Summen-Signal-Sensor jeweils ein Summen-Signal.

Im Folgenden wird die Erfindung anhand eines Ausführungsbeispiels beschrieben. Hierbei zeigt

Figur 1    einen beispielhaften Abschnitt eines kardiogenen Signals im Verlauf eines einzigen Herzschlags;

Figur 2    schematisch, welche Sensoren welche verschiedenen Größen messen, die für die Ermittlung eines geschätzten respiratorischen Signals verwendet werden;

Figur 3    einen beispielhaften Verlauf des Summen-Signals sowie beispielhaft zwei Herzschlag-Zeitpunkte und vier Atemzug-Zeiträume;

Figur 4    im Überblick den Kompensations-Funktionsblock und den Dämpfungs-Funktionsblock;

Figur 5    beispielhaft, wie unter idealen Bedingungen ein kardiogener Referenz-Signalabschnitt erzeugt wird;

Figur 6    mehrere zeitrichtig positionierte Abschnitte des Kompensations-Signals ohne sowie mit Störungen;

Figur 7    die beiden Funktionsblöcke von Figur 4, wobei die Dämpfung durch den Kompensations-Funktionsblock detaillierter dargestellt wird;

Figur 8    mehrere beispielhafte Bestandteile einer Dämpfungsfunktion;

Figur 9    durchschnittliche Leistungs-Signalabschnitte $Pow_{com,av}(1)$, ..., $Pow_{com,av}(n)$ und berechnete Schwellenwerte $\varphi(1)$, ..., $\varphi(n)$ für die n Levels (Frequenzbänder);

Figur 10    einen durchschnittlichen Leistungs-Signalabschnitt $Pow_{com,av}(5)$ für das Level Nr. 5;

Figur 11    die Referenz-Dämpfungs-Signalabschnitte für die n Levels;

Figur 12    detaillierter den Dämpfungs-Funktionsblock von Figur 4;

Figur 13    den Messwert-Aufbereiter sowie den Funktionsblock, der ein Qualitätsmaß für den Messwert-Aufbereiter berechnet;

Figur 14    eine Ausgestaltung, um eine Durchschnittskurve (Baseline) des Roh-Signals zu berechnen;

Figur 15    im Detail den Funktionsblock, der zwei Qualitätsmaße liefert;

Figur 16    beispielhafte gemittelte Leistungs-Signalabschnitte und Referenz-Dämpfungs-Signalabschnitte;

Figur 17    beispielhaft einen Referenz-Dämpfungs-Signalabschnitt und einen angepassten Dämpfungs-Signalabschnitt für einen Herzschlag-Zeitraum.

Im Ausführungsbeispiel wird die Erfindung verwendet, um automatisch eine Schätzung $Sig_{res,est}$ für ein respiratorisches Signal $Sig_{res}$ zu ermitteln, wobei das zu schätzende respiratorische Signal $Sig_{res}$ mit der eigenen Atmungsaktivität eines Patienten P korreliert und daher wenigstens näherungsweise dessen eigene Atmungsaktivität beschreibt. Diese eigene Atmungsaktivität kann durch elektrische Impulse im Körper des Patienten P ausgelöst werden, wobei der Patient P diese Impulse selbst erzeugt, die eigene Atmungsaktivität also eine spontane Atmung ist, und / oder von außen stimuliert werden, beispielsweise in einem Magnetfeld. Der Index est zeigt an, dass das respiratorische Signal $Sig_{res}$ geschätzt und nicht exakt gemessen wird.

In einer Anwendung des Ausführungsbeispiels wird der Patient P wenigstens zeitweise künstlich beatmet, und zwar durch eine unterstützende künstliche Beatmung, während das geschätzte respiratorische Signal $Sig_{res,est}$ ermittelt wird. In einer anderen Anwendung wird die Erfindung dafür benutzt, um den Patienten P und insbesondere dessen eigene Atmungsaktivität zu überwachen und zu diesem Zweck das zu schätzende respiratorische Signal $Sig_{res}$ zu verwenden, ohne dass der Patient P dabei notwendigerweise durchgehend künstlich beatmet wird.

Dieses respiratorische Signal $Sig_{res}$ lässt sich nicht direkt messen. Möglich ist, eine Messsonde im Körper des Patienten P zu positionieren und Messwerte von der Sonde zu erzeugen. Möglich ist auch, auf nicht-invasiven Wege Messwerte zu gewinnen, insbesondere dadurch, dass Elektroden auf der Haut des Patienten P Messwerte aufnehmen. In der Regel ist es weder auf invasive noch auf nicht-invasive Weise möglich, direkt die im Körper des Patienten P erzeugten Impulse zu messen, welche die Atmungsmuskulatur "ansteuern", sondern lediglich elektrische Messwerte, welche beim Kontrahieren der Muskelfasern der Atmungsmuskulatur erzeugt werden, oder die Auswirkungen solcher elektrischen Messwerte beispielsweise auf ein pneumatisches Signal. Außerdem werden die elektrischen Impulse, welche die eigene Atmungsaktivität des Patienten P hervorrufen, von elektrischen Impulsen überlagert, welche die Herzaktivität des Patienten P verursachen, genauer: welche ein Kontrahieren der Herzmuskulatur bewirken. Daher lässt sich nach einer entsprechenden Aufbereitung von Messwerten lediglich ein Summen-Signal $Sig_{Sum}$ direkt messen. Dieses Summen-Signal $Sig_{Sum}$ entsteht aus einer Überlagerung des gesuchten respiratorischen Signals $Sig_{res}$, welches mit der eigenen Atmungsaktivität des Patienten P korreliert, und eines kardiogenen Signals $Sig_{kar}$, welches mit seiner Herzaktivität korreliert. Das Summen-Signal $Sig_{Sum}$ kann von weiteren Signalen beeinflusst werden, insbesondere von solchen Signalen, die auf einen Übertragungskanal von der Signalquelle im Körper des Patienten zu einem Messort einwirken, sowie von externen Signalquellen. Diese weiteren Signale sind in der Regel Störgrößen. An diesem Messort werden die Messwerte gemessen, aus denen der Summen-Signal $Sig_{Sum}$ erzeugt wird.

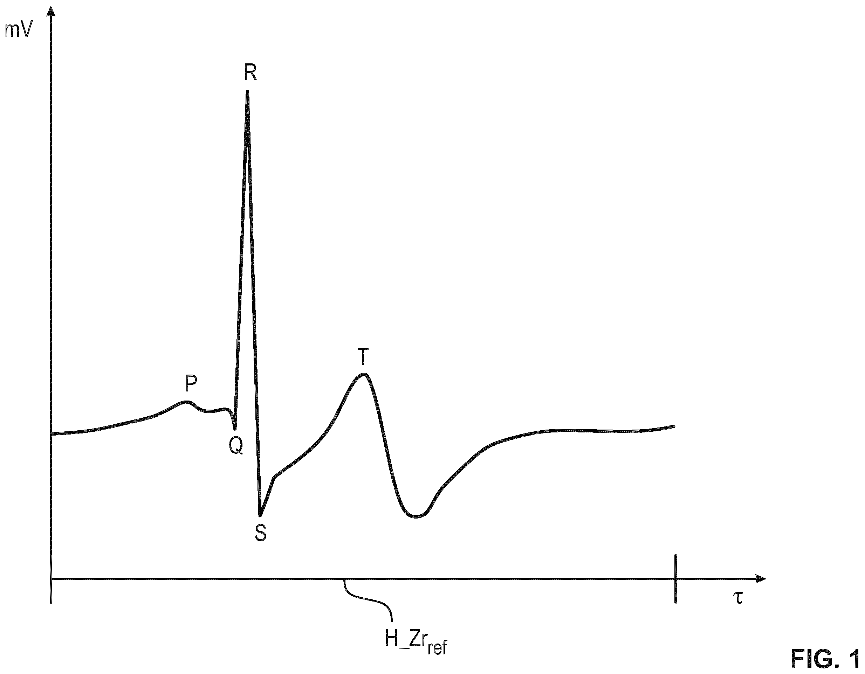

In Figur 1 wird ein typischer Abschnitt eines elektrisch gemessenen kardiogenen Signals $Sig_{kar}$ im Verlaufe eines einzigen Herzschlags gezeigt. Auf der x-Achse ist beispielhaft ein Referenz-Herzschlag-Zeitraum $H\_Zr_{ref}$ gezeigt, auf der y-Achse der Signalwert beispielsweise in Millivolt. Zu sehen sind fünf Spitzen P, Q, R, S und T. Ein charakteristischer Herzschlag-Zeitpunkt ist beispielsweise die Q-Spitze, die R-Spitze, die S-Spitze oder auch der zeitliche Mittelpunkt zwischen der Q-Spitze und der S-Spitze dieses Herzschlags oder der zeitliche Mittelpunkt zwischen der P-Spitze und der T-Spitze.

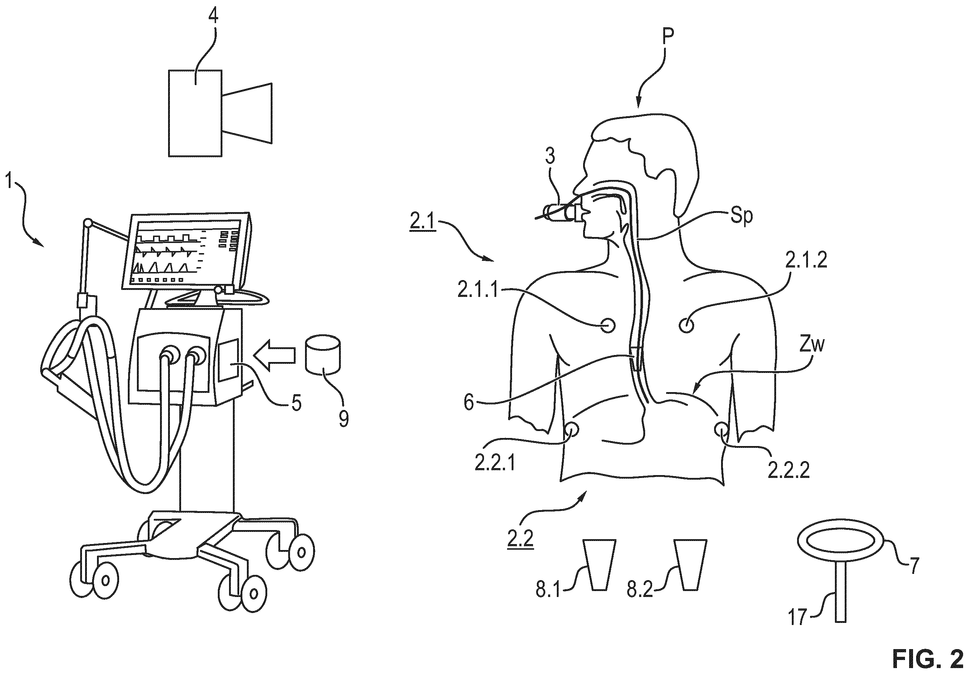

Figur 2 zeigt schematisch, welche Signale sich aus Messwerten erzeugen lassen, indem die Messwerte am und / oder im Körper des Patienten P erzeugt und auf geeignete Weise automatisch aufbereitet werden. Schematisch dargestellt werden

- der künstlich beatmete Patient P,
- die Speiseröhre Sp und das Zwerchfell Zw des Patienten P,
- ein Beatmungsgerät (ventilator) 1, welches den Patienten P zumindest zeitweise künstlich beatmet und welches eine Signalverarbeitungseinheit 5 umfasst, wobei die Signalverarbeitungseinheit 5 zumindest zeitweise Lesezugriff und Schreibzugriff auf einen Datenspeicher 9 aufweist,
- ein interkostales Paar 2.1 mit zwei Messelektroden 2.1.1 und 2.1.2, welche rechts und links vom Brustbein und zwischen jeweils zwei Rippen des Patienten P angeordnet sind, also in einem herznahen Bereich,
- ein zwerchfellnahes Paar 2.2 mit zwei weiteren Messelektroden 2.2.1 und 2.2.2, die nahe dem Zwerchfell Zw des Patienten P angeordnet sind, also in einem herzfernen Bereich,
- eine nicht gezeigte Elektrode für Masse,
- ein pneumatischer Sensor 3, der vom Körper des Patienten P räumlich entfernt ist und einen Messwert-Aufnehmer, der beispielsweise vor dem Mund des Patienten P angeordnet ist, sowie eine datenverarbeitende Auswerteeinheit, die im Beatmungsgerät 1 angeordnet sein kann, umfasst,
- ein optionaler optischer Sensor 4, der ein Bildaufnahmegerät und eine Bildauswerteeinheit umfasst und auf den Körper des Patienten P gerichtet ist,
- ein optionaler pneumatischer Sensor 6 in Form einer Sonde oder eines Ballons in der Speiseröhre Sp und nahe des Zwerchfells Zw des Patienten P,
- eine schematisch gezeigte Manschette 7 um ein Handgelenk des Patienten P, wobei diese Manschette 7 einen Katheter 17 hält, um invasiv den zeitlichen Verlauf des Blutdrucks zu messen,
- zwei Finger-Clips 8.1, 8.2, die über jeweils einen Finger des Patienten P gelegt sind oder an einer anderen Stelle auf der Haut des Patienten P positioniert sind, wobei der eine Finger-Clip 8.1 den Grad der Sättigung des Bluts mit Sauerstoff nicht-invasiv misst, bevorzugt mit Hilfe eines plethysmographischen Verfahrens, und der andere Finger-Clip 8.2 nicht-invasiv den Blutdruck des Patienten P misst, und
- optional nicht gezeigte Elektroden in der Speiseröhre des Patienten P,
- ein Bildschirm, auf dem zeitliche Verläufe von Signalen dargestellt werden.

Das interkostale Paar 2.1 und die Masse-Elektrode liefern nach Signalaufbereitung ein erstes Summen-Signal $Sig_{Sum}(1)$. Das zwerchfellnahe Paar 2.2 und die Masse-Elektrode liefern nach Signalaufbereitung ein zweites Summen-Signal $Sig_{Sum}(2)$. Die weiteren oben beschriebenen Sensoren können weitere Summen-Signale $Sig_{Sum}(n)$ liefern, $n >= 3$. Möglich ist auch, dass dieselbe Sensor-Anordnung zwei verschiedene Summen-Signale liefert, beispielsweise durch Anwendung von unterschiedlichen Messverfahren. Eine solche Sensor-Anordnung wird beispielsweise in DE 10 2009 035 018 A1 und US 2011 / 0 028 819 A1 beschrieben. Im Folgenden wird kurz von "dem Summen-Signal $Sig_{Sum}$" gesprochen.

Anstelle eines elektrischen Signals (EMG-Signal) lässt sich z.B. auch ein Summen-Signal $Sig_{Sum}$ in Form eines Mechanomyogramms (MMG-Signal) erzeugen und verwenden.

Um das Beatmungsgerät 1 während der künstlichen Beatmung des Patienten P zu regeln oder um den Patienten P zu überwachen und für die Regelung oder Überwachung das geschätzte respiratorische Signal $Sig_{res,est}$ zu verwenden, wird mit einer hohen Abtast-Frequenz das geschätzte respiratorische Signal $Sig_{res,est}$ ermittelt, d. h. zu jedem Abtast-Zeitpunkt t liefert die Signalverarbeitungseinheit 5 einen neuen Signalwert $Sig_{res,est}(t)$. Unter einer "hohen Abtast-Frequenz" wird verstanden, dass zwischen zwei aufeinanderfolgenden Abtast-Zeitpunkten ein Abstand von weniger als fünf, bevorzugt weniger als drei Millisekunden liegt. Insbesondere für die Fatigue-Ermittlung liegt die Abtastfrequenz bevorzugt bei mindestens 1 kHz, besonders bevorzugt bei mindestens 2 kHz. Einige Schritte des nachfolgend beschriebenen Verfahrens werden im Ausführungsbeispiel hingegen mit einer niedrigen Abtast-Frequenz durchgeführt, nämlich mit einer Frequenz, die im Bereich der Herzschlagfrequenz liegt, also zwischen 1 Hz und 2 Hz.

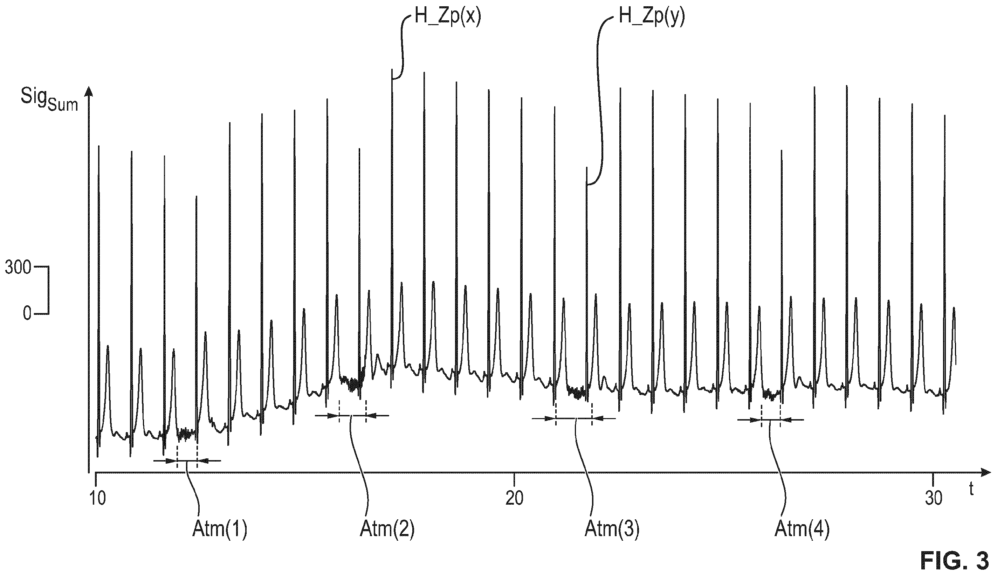

Figur 3 zeigt einen beispielhaften zeitlichen Verlauf des Summen-Signals $Sig_{Sum}$ mit vier Atemzügen und einer Vielzahl von Herzschlägen. Auf der x-Achse ist die Zeit aufgetragen, auf der y-Achse eine vom Summen-Signal-Sensor gemessene Größe, beispielsweise eine elektrische Spannung in Millivolt. Dargestellt sind die vier Zeitspannen Atm(1), ..., Atm(4) der vier Atemzüge und beispielhaft zwei charakteristische Herzschlag-Zeitpunkte H_Zp(x) und H_Zp(y). Zu sehen ist, dass das kardiogene Signal $Sig_{kar}$ in einem Herzschlag-Zeitraum H_Zp(x), H_Zp(y) um ein Vielfaches größer ist als das respiratorische Signal $Sig_{res}$ in diesem Zeitraum. Außerhalb eines Herzschlag-Zeitraums ist das respiratorische Signal $Sig_{res}$ aber im Vergleich zu dem kardiogenen Signal $Sig_{kar}$ ausreichend stark und kann daher aus dem Summen-Signal $Sig_{Sum}$ ermittelt werden.

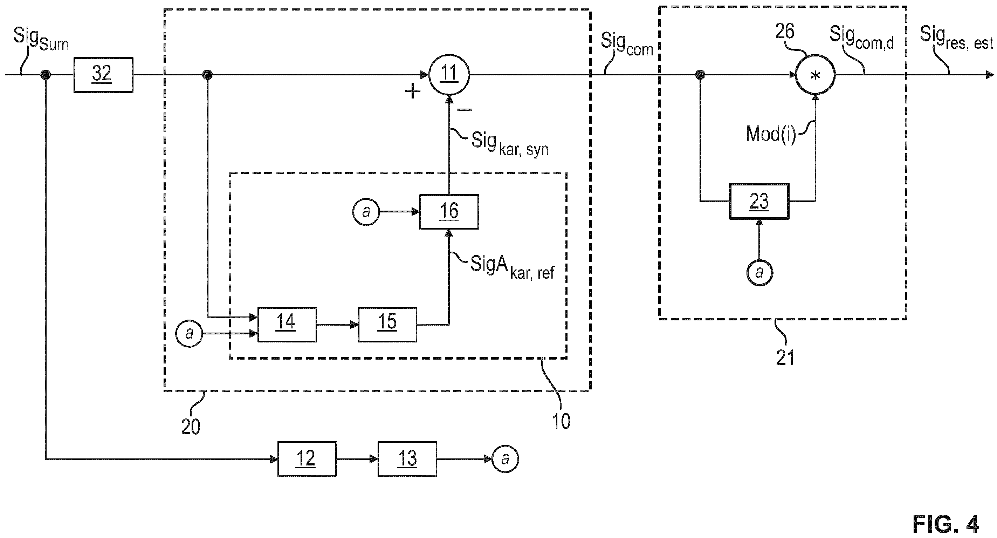

Figur 4 zeigt schematisch zwei Funktionsblöcke 20 und 21 der Signalverarbeitungseinheit 5, wobei die Funktionsblöcke 20, 21 jeweils verschiedene Signalverarbeitungs-Schritte durchführen, um rechnerisch im Summen-Signal $Sig_{Sum}$ den Einfluss der Herzaktivität $Sig_{kar}$ auf das gemessene Summen-Signal $Sig_{Sum}$ wenigstens teilweise zu kompensieren. Das Ausgangssignal eines Kompensations-Funktionsblocks 20, nämlich ein nachfolgend beschriebenes Kompensations-Signal $Sig_{com}$, liegt als Eingangssignal an einem Dämpfungs-Funktionsblock 21 an. Als Ausgangssignal liefert der Dämpfungs-Funktionsblock 21 die gesuchte Schätzung $Sig_{res,est}$.

Eine Funktionseinheit 10 des Kompensations-Funktionsblocks 20 erzeugt ein synthetisches kardiogenes Signal $Sig_{kar,syn}$, welches eine Näherung (Schätzung) für das kardiogene Signal $Sig_{kar}$ ist und aus Signalabschnitten zusammengesetzt wird (daher die Bezeichnung synthetisch). Jeder Signalabschnitt beschreibt die Herzaktivität im Verlauf eines Herzschlags. Beispielhaft wird ein solcher Signalabschnitt in Figur 1 gezeigt. Die Signalabschnitte werden zeitrichtig positioniert, optional angepasst und zum synthetischen kardiogenen Signal $Sig_{kar,syn}$ zusammengesetzt. Das synthetische kardiogene Signal $Sig_{kar,syn}$ ist eine Schätzung für das tatsächliche kardiogene Signal $Sig_{kar}$. Ein Verfahren, um einen Signalabschnitt anzupassen, wird in DE 10 2019 006 866 A1 und US 2022 / 0 330 837 A1 beschrieben.

Der Kompensations-Funktionsblock 20 kompensiert rechnerisch, beispielsweise durch Subtraktion, den Beitrag des synthetischen kardiogenen Signals $Sig_{kar,syn}$ zum Summen-Signal $Sig_{Sum}$ und erzeugt dadurch das Kompensations-Signal $Sig_{com}$, das als Zwischen-signal fungiert.

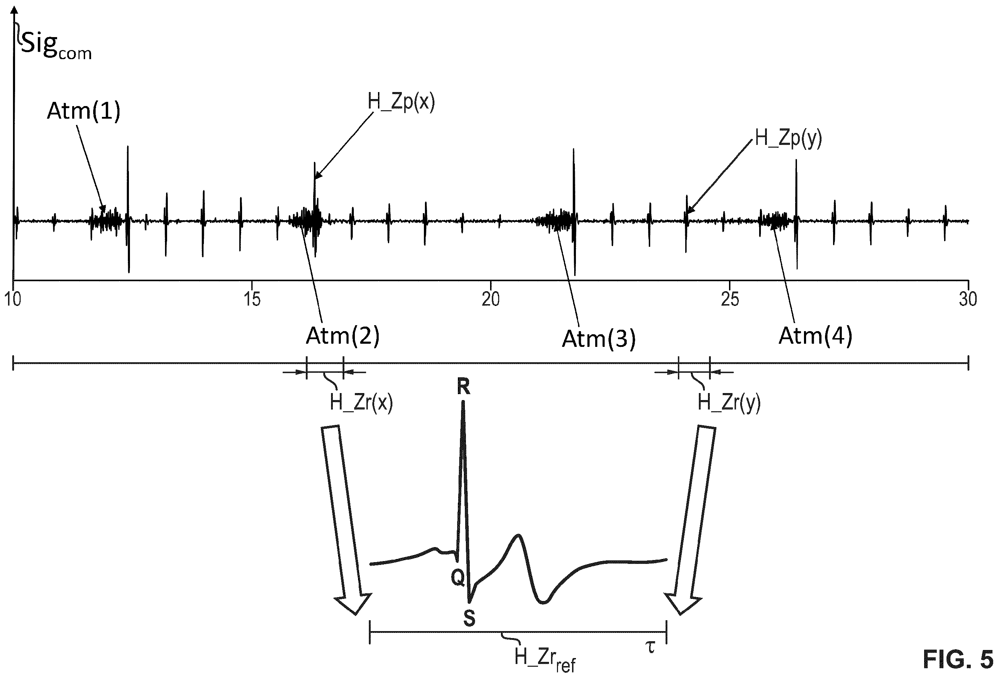

Figur 5 zeigt einen beispielhaften zeitlichen Verlauf des Kompensations-Signals $Sig_{com}$. Dieser beispielhafte zeitliche Verlauf entsteht dadurch, dass der Kompensations-Funktionsblock 20 so wie gerade beschrieben das in Figur 3 beispielhaft gezeigte Summen-Signal $Sig_{Sum}$ bearbeitet. Außerdem werden in Figur 5 zwei beispielhafte Herzschlag-Zeiträume H_Zp(x) und H_Zp(y) gezeigt. Veranschaulicht wird, wie diese beiden Herzschlag-Zeiträume H_Zp(x) und H_Zp(y) auf denselben Referenz-Herzschlag-Zeitraum $H\_Zr_{ref}$ abgebildet werden.

Der Kompensations-Funktionsblock 20 von Figur 4 erzeugt in einer Initialisierungsphase einen kardiogenen Referenz-Signalabschnitt $SigA_{kar,ref}$, der im Datenspeicher 9 abgespeichert wird, und wendet diesen in einer nachfolgenden Nutzphase für jeden detektierten Herzschlag erneut an. Folgende Schritte werden durchgeführt:

- Eine Funktionseinheit 12 identifiziert im Summen-Signal $Sig_{Sum}$ den jeweiligen Beginn und das jeweilige Ende und / oder die jeweilige QRS-Phase jedes Herzschlags, also den jeweiligen charakteristischen Herzschlag-Zeitraum H_Zr(x), H_Zr(y).

- Eine Funktionseinheit 13 ermittelt den jeweiligen genauen charakteristischen Herzschlag-Zeitpunkt H_Zp(x), H_Zp(y) jedes Herzschlags, und zwar bevorzugt mit einer Toleranz von wenigen Millisekunden. Besonders bevorzugt beträgt die Toleranz höchstens die Hälfte der Zeitspanne zwischen zwei aufeinanderfolgenden Abtast-Zeitpunkten für die Ermittlung des Summen-Signals $Sig_{Sum}$, wobei diese Zeitspanne bevorzugt unter 1 Millisekunde liegt.

- Die Funktionseinheiten 12 und 13 benötigen mehrere Werte des Summen-Signals $Sig_{Sum}$ für mehrere aufeinanderfolgende Abtast-Zeitpunkte, um den genauen Herzschlag-Zeitpunkt H_Zp(x) jedes Herzschlags zu ermitteln. In einer Ausgestaltung verzögert eine optionale Funktionseinheit 32 das Summen-Signal $Sig_{Sum}$ für die nachfolgenden Schritte um einen entsprechenden Zeitraum, vgl. Figur 4. Dadurch steht in den nachfolgenden Schritten der genaue Herzschlag-Zeitpunkt H_Zp(x) zur Verfügung. Diese optionale Funktionseinheit 32 ist in den folgenden Figuren fortgelassen. Diese Verzögerung wird nur dann angewendet, wenn die jeweilige Anwendung nicht das geschätzte respiratorische Signal $Sig_{res,est}$ in Echtzeit benötigt.

In der Initialisierungsphase werden N Herzschläge detektiert. Zu jedem Herzschlag Nr. x gehört ein Abschnitt $SigA_{Sum}(x)$ des Summen-Signals $Sig_{Sum}$. Diese N Herzschläge liefern N also Stichprobenelemente für eine Stichprobe.

In der Initialisierungsphase werden weiterhin folgende Schritte durchgeführt:

- Eine Funktionseinheit 14 überlagert rechnerisch die N Summen-Signalabschnitte $SigA_{Sum}(x_1)$, ..., $SigA_{Sum}(x_N)$ für die letzten N Herzschläge $x_1$, ..., $x_N$, und zwar zeitrichtig. Bei Bedarf werden diese N Summen-Signalabschnitte auf eine übereinstimmende Länge zugeschnitten oder gestaucht oder gestreckt.

- Bevorzugt werden die Signalabschnitte $SigA_{Sum}(x_1)$, ..., $SigA_{Sum}(x_N)$ für die N Herzschläge so überlagert, dass sie die gleiche Länge haben und die R-Spitzen oder sonstige charakteristische Herzschlag-Zeitpunkte übereinanderliegen. Jeder Signalabschnitt bezieht sich somit auf denselben Referenz-Herzschlag-Zeitraum $H\_Zr_{ref}$, vgl. Figur 5. Ein relativer Zeitpunkt in diesem Referenz-Herzschlag-Zeitraum $H\_Zr_{ref}$ wird mit $\tau$ bezeichnet. Jedem absoluten Zeitpunkt t des Summen-Signalabschnitts $SigA_{Sum}(x)$ entspricht ein relativer Zeitpunkt $\tau = \tau(t)$ in diesem relativen Herzschlag-Zeitraum. Anstelle der Bezeichnung "relativer Zeitpunkt" lässt sich auch die Bezeichnung "Herzphase $\phi$" mit einem Wertebereich von 0° bis 360° oder von 0 bis $2\pi$ verwenden.

- Eine Funktionseinheit 15 erzeugt aus der Überlagerung von N Signalabschnitten $SigA_{Sum}(x_1)$, ..., $SigA_{Sum}(x_N)$, welche die Funktionseinheit 14 erzeugt hat, einen kardiogenen Referenz-Signalabschnitt (template) $SigA_{kar,ref}$. Dieser kardiogene Referenz-Signalabschnitt $SigA_{kar,ref}$ beschreibt näherungsweise den Verlauf des kardiogenen Signals $Sig_{kar}$ während eines einzigen Herzschlags und bezieht sich ebenfalls auf den Referenz-Herzschlag-Zeitraum $H\_Zr_{ref}$. Bevorzugt liegt der charakteristische Herzschlag-Zeitpunkt $H\_Zp(x)$ bei $\tau=0$. Wie bereits erwähnt, ist während eines Herzschlags der kardiogene Anteil im Summen-Signal $Sig_{Sum}$ um ein Vielfaches größer als der respiratorische Anteil, und durch die Mittelung über N Signalabschnitte werden die respiratorischen Anteile während eines Herzschlag-Zeitraums weitgehend "herausgemittelt".

- Die Funktionseinheit 15 wendet bevorzugt auf die N Signalabschnitte für die jeweils letzten N Herzschläge ein lernendes Verfahren an. Der kardiogene Referenz-Signalabschnitt $SigA_{kar,ref}$ wird bevorzugt im Datenspeicher 9 abgespeichert. Dieser kardiogene Referenz-Signalabschnitt $SigA_{kar,ref}$ bezieht sich auf den Patienten P und auf dessen aktuellen Zustand, ist also nicht eine Mittelung über Signale von verschiedenen Patienten.

In einer nachfolgenden Nutzphase werden folgende Schritte durchgeführt:

- Die Funktionseinheiten 32 und 13 detektieren im Summen-Signal $Sig_{Sum}$ die Herzschläge und ermitteln den jeweiligen charakteristischen Herzschlag-Zeitpunkt jedes detektierten Herzschlags.

- Für jeden Herzschlag Nr. x wird erneut der kardiogene Referenz-Signalabschnitt $SigA_{kar,ref}$ verwendet. In einer Ausgestaltung wird dieser unverändert vom Summen-Signalabschnitt $SigA_{Sum}(x)$ für den Herzschlag-Zeitraum $H\_Zr(x)$ des Herzschlags x subtrahiert (template subtraction).

- Optional verwendet eine Funktionseinheit 16 hingegen den Wert mindestens eines anthropologischen Parameters, welcher die Herzaktivität und damit das kardiogene Signal $Sig_{kar}$ beeinflusst und bei diesem Herzschlag Nr. x gemessen worden ist. Der Lungen-Füllstand und ein Maß für die aktuelle Körperhaltung des Patienten P sowie der Abstand RR zwischen den R-Spitzen von zwei aufeinanderfolgenden Herzschlägen sind Beispiele für einen solchen anthropologischen Parameter. Die Funktionseinheit 16 passt für jeden Herzschlag den kardiogenen Referenz-Signalabschnitt $SigA_{kar,ref}$ an den oder jeden bei diesem Herzschlag gemessenen anthropologischen Parameter-Wert an und erzeugt dadurch einen kardiogenen Signalabschnitt $SigA_{kar}(x)$. Ein derartiges Vorgehen wird beispielsweise in DE 10 2019 006 866 A1 (US 2022 / 0 330 837 A1) und DE 10 2020 002 572 A1 (US 2021 / 0 338 176 A1) beschrieben.

- Die Funktionseinheit 16 positioniert zeitrichtig, z.B. QRS-synchronisiert, den kardiogenen Referenz-Signalabschnitt $SigA_{kar,ref}$ oder optional den angepassten kardiogenen Signalabschnitt $SigA_{kar}(x)$ relativ zum Summen-Signalabschnitt $SigA_{Sum}(x)$ des aktuellen Herzschlags Nr. x. Dadurch wird ein neuer synchronisierter Abschnitt $SigA_{kar,syn}(x)$ des synthetischen kardiogenen Signals $Sig_{kar,syn}$ generiert. Bevorzugt wird das synthetische kardiogene Signal $Sig_{kar,syn}$ in einer von einem Menschen wahrnehmbaren Form ausgegeben, beispielsweise auf der Bildschirmeinheit von Figur 2.

- Eine Funktionseinheit 11 kompensiert im neuesten Summen-Signalabschnitt $SigA_{Sum}(x)$ den Einfluss des kardiogenen Signals $Sig_{kar}$, beispielsweise indem die Funktionseinheit 11 vom neuesten Summen-Signalabschnitt $SigA_{Sum}(x)$ den kardiogenen Referenz-Signalabschnitt $SigA_{kar,ref}$ oder den angepassten kardiogenen Signalabschnitt $SigA_{kar}(x)$ subtrahiert. Das Ergebnis ist ein neuer Abschnitt $SigA_{com}(x)$ des Kompensations-Signals $Sig_{com}$.

Zu Beginn des Verfahrens, also nachdem der Patient P mit den Messelektroden 2.1.1 bis 2.2.2 verbunden ist, wird die Initialisierungsphase durchgeführt, welche einen Zeitraum von N detektierten Herzschlägen umfasst. In dieser Initialisierungsphase generiert der Kompensations-Funktionsblock 20 so wie oben beschrieben abhängig von den Summen-Signalabschnitten $SigA_{Sum}(x_1)$, ... , $SigA_{Sum}(x_N)$ für die letzten N Herzschläge einen initialen kardiogenen Referenz-Signalabschnitt $SigA_{kar,ref}$. Während des Verfahrens passt der Kompensations-Funktionsblock 20 den kardiogenen Referenz-Signalabschnitt $SigA_{kar,ref}$ bevorzugt an die jeweils letzten N Herzschläge an und speichert das Ergebnis im Datenspeicher 9 ab. Die Schritte in der Initialisierungsphase und die Anpassung an die jeweils letzten N Herzschläge werden mit der niedrigen Abtast-Frequenz, die etwa gleich der Herzschlagfrequenz ist, durchgeführt.

Bevorzugt werden die Abschnitte für einen Herzschlag mit der doppelten Zeitauflösung des Summen-Signals $Sig_{Sum}$ überlagert. Dies bedeutet: Die Werte des Summen-Signals $Sig_{Sum}$ werden mit einer hohen Abtast-Frequenz f ermittelt, d.h. der Abstand $\Delta t$ zwischen zwei Abtast-Zeitpunkten beträgt 1/f. Rechnerisch wird die Zeitauflösung auf z.B. 2f oder 3f vergrößert, z.B. indem zwischen zwei aus Messwerten hergeleiteten Signalwerten $Sig_{Sum}(t)$ und $Sig_{Sum}(t+\Delta t)$ jeweils rechnerisch ein Signalwert $Sig_{Sum}(t+\Delta t/2)$ positioniert wird, beispielsweise durch Interpolation.

Nach der Initialisierungsphase werden die folgenden Schritte mit der hohen Abtast-Frequenz (wenige Millisekunden oder sogar nur wenige Zehntel-Millisekunden) durchgeführt:

- Die Signalverarbeitungseinheit 5 leitet aus Messwerten jeweils einen neuen Wert $Sig_{Sum}(t)$ für das Summen-Signal $Sig_{Sum}$ her.
- Die Funktionseinheiten 12 und 13 erkennen im Summen-Signal $Sig_{Sum}$ den Beginn des Herzschlag-Zeitraums $H\_Zr(x)$ bzw. den genauen charakteristischen Zeitpunkt $H\_Zp(x)$ eines Herzschlags x und ermitteln dadurch einen neuen Summen-Signalabschnitt $SigA_{Sum}(x)$.
- Der Kompensations-Funktionsblock 20 passt optional den kardiogenen Referenz-Signalabschnitt $SigA_{kar,ref}$ an den jeweiligen Wert mindestens eines anthropologischen Parameters an. Der Kompensations-Funktionsblock 20 ermittelt den zugeordneten relativen Zeitpunkt $\tau = \tau(t)$ und generiert durch zeitrichtige Positionierung einen weiteren Signalabschnitt, nämlich den zeitlich neuesten Abschnitt $SigA_{kar,syn}(x)$ des synthetischen kardiogenen Signals $Sig_{kar,syn}$.
- Die Funktionseinheit 11 subtrahiert vom neuen Wert $Sig_{Sum}(t)$ den Wert $SigA_{kar,ref}[\tau(t)]$ bzw. $SigA_{kar}(x)$ $[T(t)]$ des kardiogenen Referenz-Signalabschnitts $SigA_{kar,ref}$ oder des angepassten kardiogenen Signalabschnitts $SigA_{kar}(x)$ für denselben relativen Zeitpunkt $\tau$, also

$$Sig_{com}(t) = Sig_{Sum}(t) - SigA_{kar,syn}[\tau(t)]$$

oder kompensiert auf andere Weise den kardiogenen Einfluss.
- Der Kompensations-Funktionsblock 20 gibt einen neuen Signalabschnitt $SigA_{com}(x)$ für das Kompensations-Signal $Sig_{com}$ aus.

Idealerweise enthält das Kompensations-Signal $Sig_{com}$ alle Beiträge der Herzaktivität $Sig_{kar}$ zum Summen-Signal $Sig_{Sum}$. In der Praxis ist dies nicht der Fall. Hierfür gibt es vor allem folgende beiden möglichen Ursachen:

- Mögliche Störungen in der Initialisierungsphase führen zu einem kardiogenen Referenz-Signalabschnitt $SigA_{kar,ref}$, der in relevanter Weise von der Realität abweicht.
- Mögliche Störungen in der Nutzphase beeinflussen das Summen-Signal $Sig_{Sum}$.

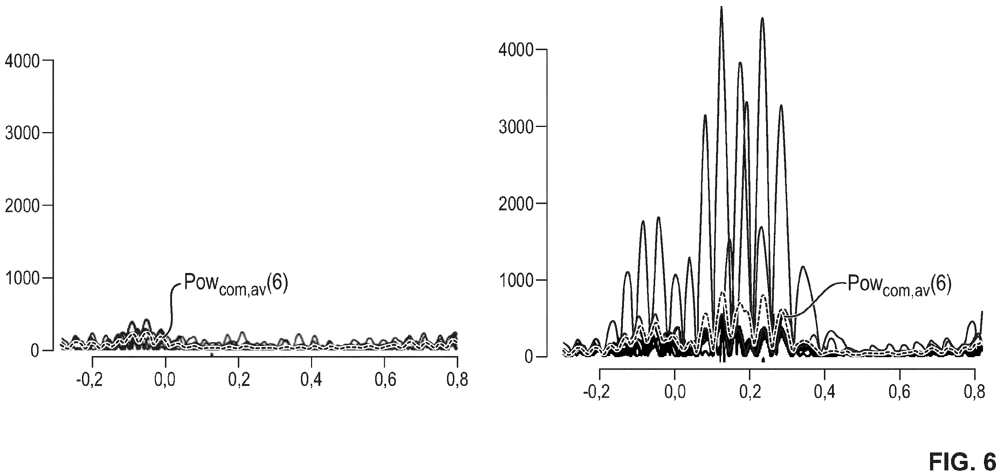

Figur 6 veranschaulicht die Auswirkungen von möglichen Störungen. In beiden Diagrammen sind jeweils mehrere Signalabschnitte für den zeitlichen Verlauf einer elektrischen Leistung des Signals gezeigt, darunter der Signalabschnitt $Pow_{com,av}(6)$, der weiter unten erläutert wird. Jeder Signalabschnitt deckt einen einzigen Herzschlag-Zeitraum $H\_Zr(x)$, $H\_Zr(y)$ ab. Im linken Diagramm treten keine Störungen auf, während im rechten Diagramm mehrere Störungen auftreten, die zu großen Oszillationen des Kompensations-Signals $Sig_{com}$ führen. Diese Störungen sind beispielsweise beim Erzeugen der Messwerte aufgetreten. Diese großen Oszillationen können ohne eine geeignete Gegenmaßnahme zu fehlerhaften Ergebnissen führen.

Im Ausführungsbeispiel wird ein Dämpfungs-Funktionsblock 21 angewendet, um das Kompensations-Signal $Sig_{com}$ nachzubearbeiten, vgl. Figur 4. Das Ausgangssignal des Kompensations-Funktionsblocks 20, nämlich das Kompensations-Signal $Sig_{com}$, liegt als Eingangssignal am Dämpfungs-Funktionsblock 21 an.

Eine Funktionseinheit 23 des Dämpfungs-Funktionsblocks 21 erzeugt aus dem Kompensations-Signal $Sig_{com}$ einen weiter unten beschriebenen Dämpfungs-Signalabschnitt $Mod(i)$. Eine Funktionseinheit 26 wendet diesen Dämpfungs-Signalabschnitt $Mod(i)$ auf das Kompensations-Signal $Sig_{com}$ an, bewirkt dadurch rechnerisch eine Verringerung der elektrischen Leistung, insbesondere eine Dämpfung, und erzeugt dadurch das geschätzte respiratorische Signal $Sig_{res,est}$.

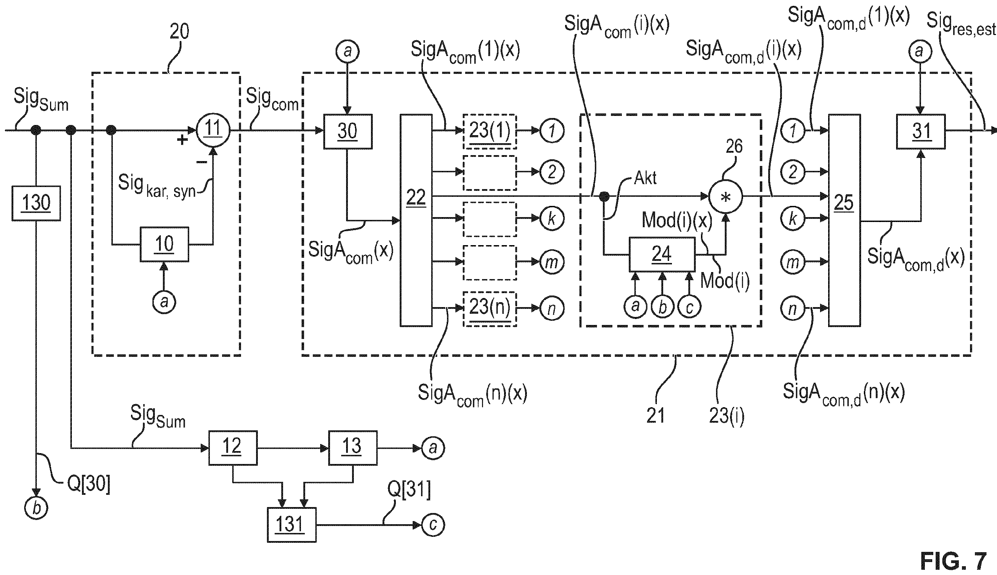

Der Dämpfungs-Funktionsblock 21 wird nachfolgend mit Bezug auf Figur 7 näher beschrieben. Am Dämpfungs-Funktionsblock 21 liegt das Kompensations-Signal $Sig_{com}$ an.

Vorgegeben werden n Frequenzbänder, die bei einer Wavelet-Transformation auch "Levels" genannt werden. Hierbei ist n eine vorgegebene Anzahl. Bevorzugt liegt n zwischen 5 und 10 und beträgt besonders bevorzugt 8. Das Level 1 gehört zu dem Frequenzband mit den höchsten Frequenzen, das Level n zu dem Frequenzband mit den niedrigsten Frequenzen.

Soweit nicht anders erwähnt, bezieht sich die nachfolgende Beschreibung auf die Nutzphase.

Eine Funktionseinheit 30 erzeugt aus dem Kompensations-Signal $Sig_{com}$ den Kompensations-Signalabschnitt $SigA_{com}(x)$ für den neuesten detektierten Herzschlag x. Hierfür verwendet sie den charakteristischen Herzschlag-Zeitpunkt H_Zp(x) und den Herzschlag-Zeitraum H_Zr(x), den die Funktionseinheiten 12 und 13 unter Verwendung des Summen-Signals $Sig_{Sum}$ detektiert haben.

Eine Funktionseinheit 22 zerlegt den Kompensations-Signalabschnitt $SigA_{com}(x)$ des Kompensations-Signals $Sig_{com}$ in n Signalanteil-Abschnitte $SigA_{com}(1)(x), ..., SigA_{com}(n)(x)$ für die n Level (Frequenzbänder). Bevorzugt führt die Funktionseinheit 22 eine Wavelet-Transformation, bevorzugt eine stationäre Wavelet-Transformation oder eine transformation à trous, durch. Werden die Signalanteil-Abschnitte $SigA_{com}(i)(x)$ zeitrichtig aneinandergefügt (zusammengesetzt), so entsteht ein Signalanteil $Sig_{com}(i)$ (i = 1, ..., n).

Der Dämpfungs-Funktionsblock 21 umfasst die Funktionseinheit 22 für die Zerlegung, eine Funktionseinheit 25 für die Rücktransformation sowie für jedes Level i jeweils eine Funktionseinheit 23(i) und zwei Funktionseinheiten 24 = 24(i) und 26 = 26(i). In Figur 7 werden nur eine Funktionseinheit 24 und eine Funktionseinheit 26 gezeigt, nämlich für das Level i.

In der Initialisierungsphase erzeugt die Funktionseinheit 23(i) für jedes Level i (i = 1, ..., n) jeweils einen Referenz-Dämpfungs-Signalabschnitt Mod(i), insgesamt also n Referenz-Dämpfungs-Signalabschnitte Mod(1), ..., Mod(n). Jeder Referenz-Dämpfungs-Signalabschnitt Mod(i) beschreibt einen zeitlichen Verlauf und deckt den Referenz-Herzschlag-Zeitraum $H\_Zr_{ref}$ ab. Jeder Signalwert $Mod(i)(\tau)$ ist eine Zahl zwischen 0 und 1 (einschließlich). Für jedes Level i wird in der Initialisierungsphase also jeweils ein Referenz-Dämpfungs-Signalabschnitt Mod(i) generiert. Diese n Referenz-Dämpfungs-Signalabschnitte Mod(1), ..., Mod(n) werden im Datenspeicher 9 abgespeichert und in der Nutzphase verwendet.

Für jedes Level i wird in der Initialisierungsphase also jeweils ein Referenz-Dämpfungs-Signalabschnitt Mod(i) generiert. Der Pfeil Akt im Block 23(i) in Figur 7 deutet an, dass in einer Ausgestaltung auch in der Nutzphase der Referenz-Dämpfungs-Signalabschnitt Mod(i) laufend aktualisiert wird. Wie dies geschieht, wird weiter unten beschrieben.

In der Nutzphase wird für den Herzschlag x und für jedes Level i die jeweilige Funktionseinheit 23(i) auf den Signalanteil-Abschnitt $SigA_{com}(i)(x)$ angewendet, i = 1, ..., n. Die Funktionseinheit 24 = 24(i) der Funktionseinheit 23(i) erzeugt aus dem Referenz-Dämpfungs-Signalabschnitt Mod(i) einen angepassten Dämpfungs-Signalabschnitt Mod(i)(x), wobei der angepasste Dämpfungs-Signalabschnitt Mod(i)(x) einen zeitlichen Verlauf darstellt und den Herzschlag-Zeitraum H_Zr(x) abdeckt und wobei jeder Signalwert Mod(i)(x)(t) eine Zahl zwischen 0 und 1 (einschließlich) ist.

Eine Funktionseinheit 26 = 26(i) wendet in der Nutzphase den zeitrichtig positionierten angepassten Dämpfungs-Signalabschnitt Mod(i)(x) auf den Signalanteil-Abschnitt $SigA_{com}(i)(x)$ für den Herzschlag x an und erzeugt den gedämpften Signalanteil-Abschnitt $SigA_{com,d}(i)(x)$ (i = 1, ..., n). Beispielsweise multipliziert die Funktionseinheit 26 die beiden Signalwerte $SigA_{com}(i)(x)(t)$ und $Mod(i)(x)[\tau(t)]$ miteinander und erzeugt dadurch für jeden Abtast-Zeitpunkt t einen Wert $SigA_{com,d}(i)(x)(t)$ des gedämpften Signalanteil-Abschnitts $SigA_{com,d}(i)(x)$, beispielsweise gemäß der Rechenvorschrift

$$SigA_{com,d}(i)(x)(t) = SigA_{com}(i)(x)(t) * Mod(i)(x)(t).$$

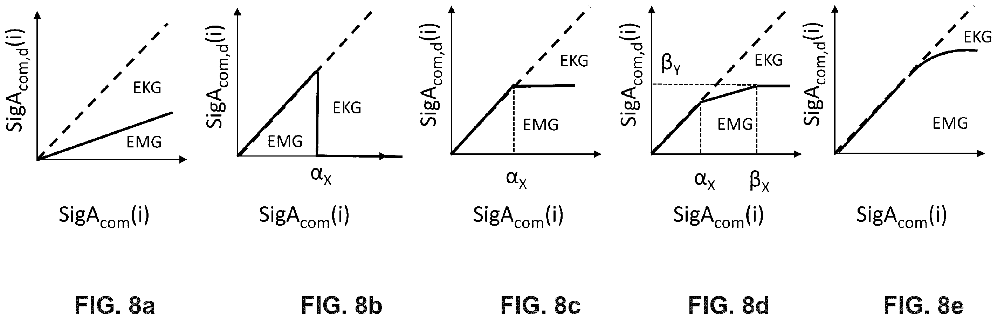

Weitere mögliche Realisierungsformen werden weiter unten mit Bezug auf Figur 8 beschrieben.

Diese Modifizierung liefert eine Dämpfung $SigA_{com,d}(i)(x)$ des Signalanteil-Abschnitts $SigA_{com}(i)(x)$. Das Vorzeichen jedes Signalwerts wird bei der Dämpfung beibehalten. Alternative Ausgestaltungen der Dämpfung werden weiter unten beschrieben.

Figur 8 veranschaulicht fünf alternative Möglichkeiten, wie der gedämpfte Signalanteil-Abschnitt $SigA_{com,d}(i)(x)$ durch Dämpfung aus dem Signalanteil-Abschnitt $SigA_{com}(i)(x)$ erzeugt wird. Durch die Dämpfung wird der Signalanteil-Abschnitt $SigA_{com}(i)(x)$ in einen respiratorischen Anteil $SigA_{com,d}(i)$, der in Figur 8 auch mit EMG bezeichnet wird, und einen kardiogenen Anteil, der als EKG bezeichnet wird, aufgeteilt.

Die Möglichkeit a) ist die gerade dargelegte Ausgestaltung, mit einem Faktor Mod(i)(x) zu multiplizieren, wobei die Steigung Mod(i)(x)(t) der Gerade von t abhängt. Die Möglichkeit b) bedeutet einen harten Schwellenwert (hard threshold) $\alpha$, wobei dieser Schwellenwert $\alpha = \alpha(t)$ ebenfalls von t abhängt. Die Möglichkeit c) bedeutet einen weichen Schwellenwert (soft threshold). Die Möglichkeit d) ist eine Mischform. Die Möglichkeit e) wird weiter unten beschrieben.

Durch die Dämpfung wird also ein gedämpfter Signalanteil-Abschnitt $SigA_{com,d}(i)(x)$ erzeugt, der sich auf den Zeitraum H_Zr(x) des letzten Herzschlags Nr. x und auf das Level Nr. i bezieht.

Die Funktionseinheit 25 setzt die gedämpften Signalanteil-Abschnitte $SigA_{com,d}(1)(x), ..., SigA_{com,d}(n)(x)$ zu einem gedämpften Signalanteil-Abschnitt $SigA_{com,d}(x)$ zusammen, wobei die Funktionseinheit 25 bevorzugt eine Wavelet-Rücktransformation (inverse Wavelet-Transformation) durchführt, und gibt diesen gedämpften Signalanteil-Abschnitt $SigA_{com,d}(x)$ als Ausgangssignal aus.

Die Funktionseinheit 31 erzeugt das gesuchte geschätzte respiratorische Signal $Sig_{res,est}$. Hierfür verwendet sie die charakteristischen Herzschlag-Zeitpunkte H_Zp(x), die Herzschlag-Zeiträume H_Zr(x) und die gedämpften Signalanteil-Abschnitte $SigA_{com,d}(x)$. Für einen Abschnitt, der zwischen zwei aufeinanderfolgenden Herzschlag-Zeiträumen H_Zr(x) und H_Zr(x+1) liegt, verwendet die Funktionseinheit 31 bevorzugt den entsprechenden Abschnitt des Kompensations-Signals $Sig_{com}$ als Abschnitt des geschätzten respiratorischen Signals $Sig_{res,est}$. Die Funktionseinheit 31 gibt das auf diese Weise erzeugte geschätzte respiratorische Signal $Sig_{res,est}$ aus.

Die Funktionseinheiten 14 und 15, die in Figur 4 gezeigt werden, aktualisieren den kardiogenen Referenz-Signalabschnitt $SigA_{kar,ref}$, sobald ein weiterer Herzschlag abgeschlossen ist, d.h. sie erzeugen einen angepassten kardiogenen Signalabschnitt $SigA_{kar}(x)$. Außerdem passt die Funktionseinheit 23(i) die Referenz-Dämpfungs-Signalabschnitte Mod(i) für die n Level an und erzeugt dadurch den angepassten Dämpfungs-Signalabschnitt Mod(i)(x), und zwar bevorzugt, sobald der weitere Herzschlag abgeschlossen ist (i = 1, ..., n).

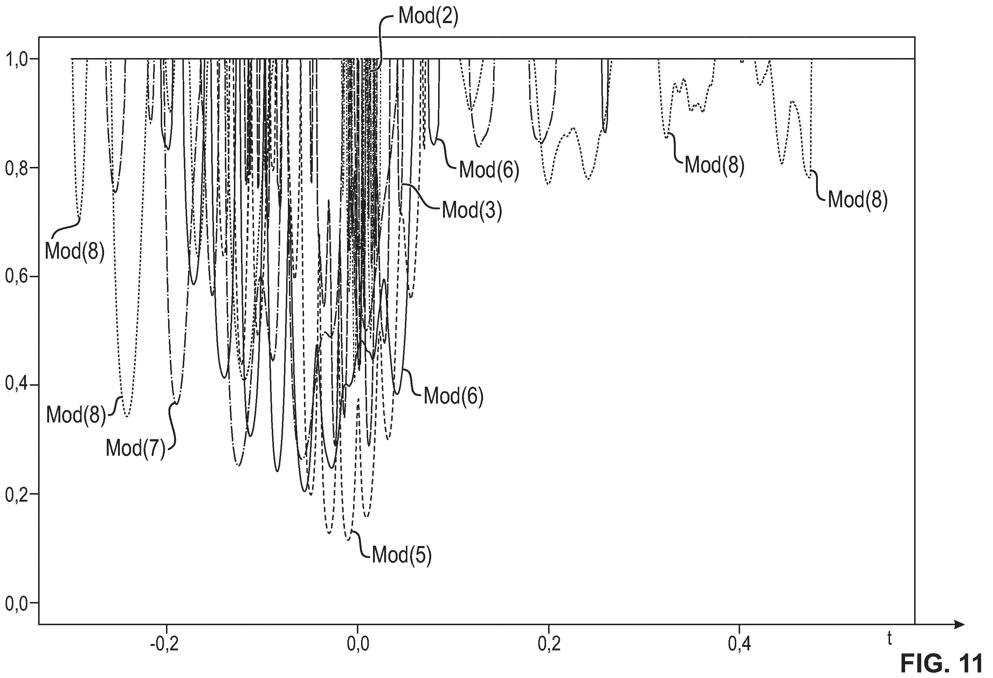

Nachfolgend wird beschrieben, wie in der Initialisierungsphase die n Referenz-Dämpfungs-Signalabschnitte Mod(1), ..., Mod(n) erzeugt werden. Figur 11 zeigt beispielhaft acht Referenz-Dämpfungs-Signalabschnitte Mod(1), ..., Mod(8), d.h. n=8. In einer Ausgestaltung führt die Funktionseinheit 24 in der Initialisierungsphase für jedes Level i und für jeden Signalanteil-Abschnitt $SigA_{com}(i)(x)$ eines Herzschlags x (i = 1, ..., n) jeweils die nachfolgend beschriebenen Schritte durch:

Die Funktionseinheit 24 ermittelt in der Initialisierungsphase einen durchschnittlichen Signalabschnitt $Pow_{com,av}(i)$ für den zeitlichen Verlauf einer elektrischen Leistung, wobei der Leistungs-Signalabschnitt $Pow_{com,av}(i)$ den Referenz-Herzschlag-Zeitraum $H\_Zr_{ref}$ abdeckt und sich auf das Level Nr. i bezieht. Der durchschnittliche Leistungs-Signalabschnitt $Pow_{com,av}(i)$ wird als ein gewichtetes Mittel über die Leistungswerte der M Signalanteil-Abschnitte $SigA_{com}(i)(x)$ von M Herzschlägen x berechnet. Beispielsweise ist

$$Pow_{com}(i)(x)(\tau) = Abs[SigA_{com}(i)(\tau)] \text{ (der Absolutwert) oder}$$

$$Pow_{com}(i)(x)(\tau) = RMS[SigA_{com}(i)(\tau)] \text{ (root mean square, RMS, der Effektivwert).}$$

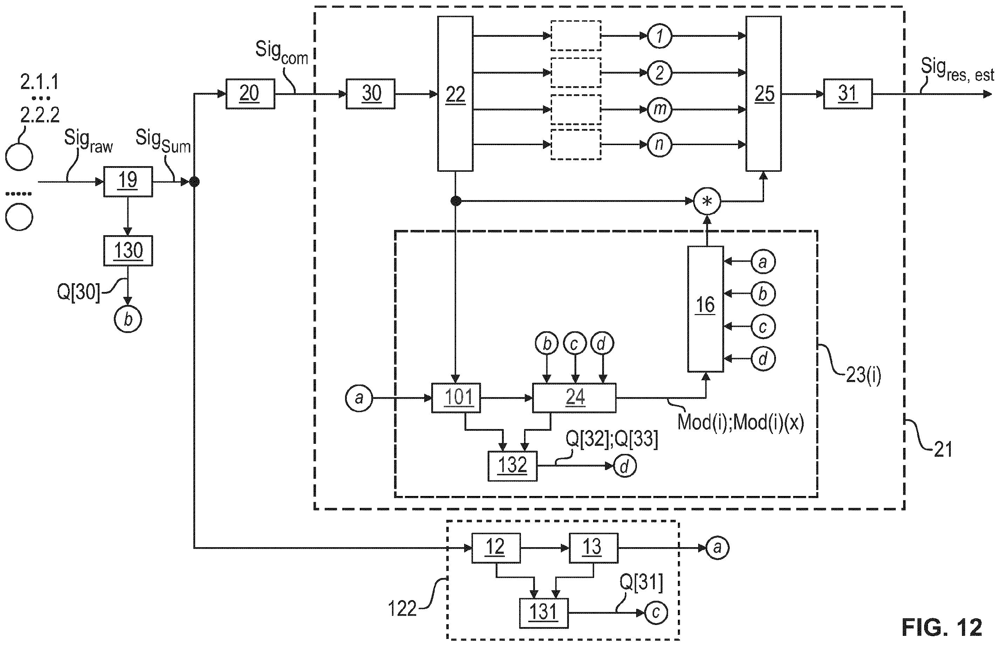

Figur 6 zeigt beispielhaft den durchschnittlichen Signalabschnitt $Pow_{com,av}(6)$ für das Level Nr. 6, der als arithmetisches Mittel berechnet wurde. Weiter unten wird mit Bezug auf Figur 12 erläutert, wie die Funktionseinheit 24 die Gewichtsfaktoren für die gewichteten Mittel bildet.

In der Initialisierungsphase werden dadurch für die M Herzschläge x M Leistungs-Signalabschnitte $Pow_{com}(i)(x)$ berechnet. Die Anzahlen M und N (Anzahl der Herzschläge zur Berechnung des kardiogenen Referenz-Signalabschnitts $SigA_{kar,ref}$) können gleich sein oder sich voneinander unterscheiden. Bevorzugt wird jeder Leistungs-Signalabschnitt $Pow_{com}(i)(x)$ unter Verwendung eines geeigneten Filters berechnet, wobei über Werte des Kompensations-Signals $Sig_{com}$ geeignet geglättet wird.

Jeder Leistungs-Signalabschnitt $Pow_{com}(i)(x)$ eines Herzschlags x überdeckt jeweils einen Herzschlag-Zeitraum H_Zr(x). Die Funktionseinheit 24 überlagert die M Leistungs-Signalabschnitte $Pow_{com}(i)(x)$ herzschlag-synchron (zeitrichtig) zu den M Herzschlägen und bildet anschließend ein gewichtetes Mittel über die überlagerten M Abschnitte. Dadurch wird ein durchschnittlicher Leistungs-Signalabschnitt $Pow_{com,av}(i)$ für das Level Nr. i ermittelt, der ein Maß für die durchschnittliche elektrische Leistung des Kompensations-Signals $Sig_{com}(i)$ im Level Nr. i während des Referenz-Herzschlag-Zeitraums $H\_Zr_{ref}$ ist, wobei die ermittelte durchschnittliche elektrische Leistung von dem relativen Zeitpunkt $\tau$ abhängt. Durch die Mittelung werden Einflussfaktoren, die nicht von der Herzaktivität des Patienten P herrühren, sondern von der Atmungsaktivität, beispielsweise von einem Husten, "hinweggemittelt".

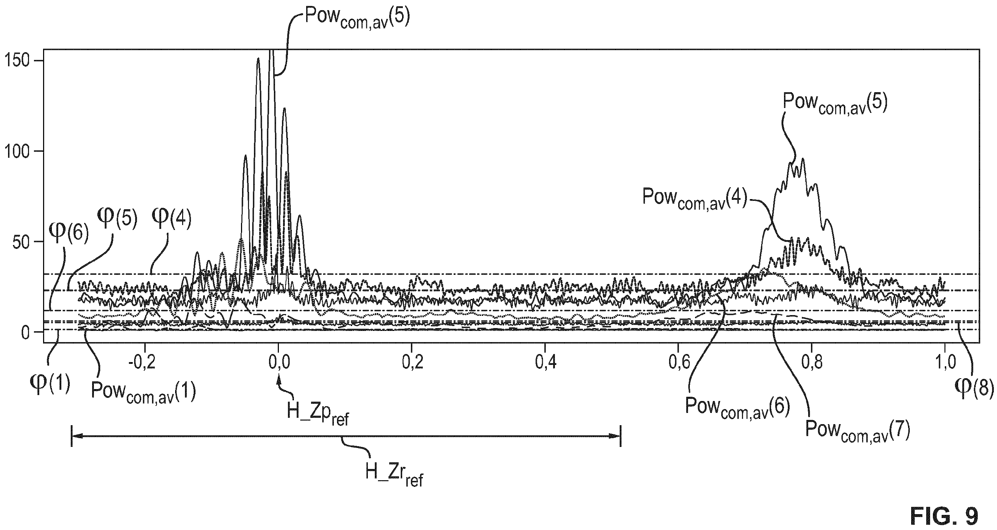

Figur 9 zeigt den Referenz-Herzschlag-Zeitraum $H\_Zr_{ref}$ sowie den Referenz-Herzschlag-Zeitpunkt $H\_Zp_{ref}$ dieses durch herzschlag-synchrone Überlagerung erzeugten durchschnittlichen Leistungs-Signalabschnitts $Pow_{com,av}(i)$. In diesem Beispiel werden acht verschiedene Levels unterschieden, also n = 8. Der Zeitpunkt t = 0 auf der x-Achse wurde in den Referenz-Herzschlag-Zeitpunkt $H\_Zr_{ref}$ gelegt. Weiterhin zeigt Figur 9 die n = 8 durchschnittlichen Leistungs-Signalabschnitte $Pow_{com,av}(1)$, ..., $Pow_{com,av}(8)$ für die n = 8 Levels.

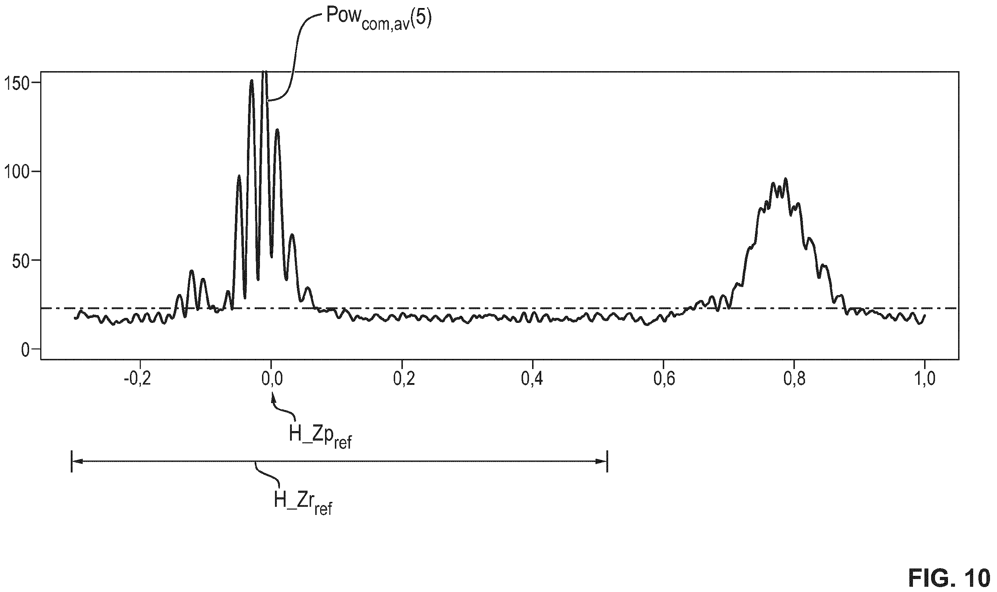

Figur 10 zeigt den durchschnittlichen Leistungs-Signalabschnitt $Pow_{com,av}(5)$ für das Level Nr. 5.

Aus dem durchschnittlichen Leistungs-Signalabschnitt $Pow_{com,av}(i)$ für das Level Nr. i werden ein mittlerer Signalwert Avg(i) und unter Verwendung des mittleren Signalwerts Avg(i) ein Schwellenwert (threshold) $\varphi(i)$ hergeleitet. Der mittlere Signalwert Avg(i) und der Schwellenwert $\varphi(i)$ variieren in der Regel von Level i1 zu Level i2 und auch für ein einziges Level i von Herzschlag zu Herzschlag, wenn der mittlere Signalwert Avg(i) und der Schwellenwert $\varphi(i)$ laufend abhängig von den jeweils letzten M Herzschlägen aktualisiert werden. Mithilfe dieses vom Kompensations-Signal $Sig_{com}$ abhängenden Schwellenwerts $\varphi(i)$ wird später ein Rauschen im Kompensations-Signal $Sig_{com}$ wenigstens teilweise rechnerisch eliminiert, wobei dieses Rauschen im Wesentlichen durch das kardiogene Signal $Sig_{kar}$ erzeugt wird. Dank des gerade beschriebenen Vorgehens werden die Schwellenwerte $\varphi(1)$, ..., $\varphi(n)$ zur Laufzeit berechnet und brauchen nicht vorgegeben zu werden.

Der mittlere Signalwert Avg(i) wird beispielsweise als arithmetisches Mittel oder auch als Median über R Signalwerte des durchschnittlichen Leistungs-Signalabschnitts $Pow_{com,av}(i)$ zu R aufeinanderfolgenden relativen Abtast-Zeitpunkten $\tau_1$, ..., $\tau_R$ des Referenz-Herzschlag-Zeitpunkts $H\_Zr_{ref}$ berechnet. Der Median ist weniger sensitiv gegenüber Ausreißern als das arithmetische Mittel, seine Berechnung erfordert aber mehr Rechenzeit.

Um den Schwellenwert $\varphi(i)$ zu berechnen, wird ein Faktor $\alpha$ vorgegeben, beispielsweise $\alpha = 2$. Der Schwellenwert $\varphi(i)$ wird beispielsweise gemäß der Rechenvorschrift

$$\varphi(i) = [1 + (n-i) / \alpha * n] * Avg(i).$$

berechnet.

Figur 9 zeigt weiterhin die n Schwellenwerte $\varphi(1)$ , ..., $\varphi(n)$ für die n Level.

Ein Signalwert $SigA_{com}(i)(x)(t)$ des Signalanteils $SigA_{com}(i)(x)$ des Kompensations-Signals $Sig_{com}$ soll umso stärker gedämpft werden, je größer der Signalwert $Pow_{com,av}(i)(\tau)$ des durchschnittlichen Leistungs-Signalabschnitts $Pow_{com,av}(i)$ zu dem entsprechenden relativen Zeitpunkt $\tau(t)$ des Referenz-Herzschlag-Zeitraums $H\_Zr_{ref}$ ist. Denn große Signalwerte stammen aufgrund der Mittelung über N Herzschlag-Zeiträume vom kardiogenen Signal $Sig_{kar}$. Die Dämpfung hängt also vom aktuell ermittelten Summen-Signal $Sig_{Sum}$ ab und nicht von einem vorgegebenen Schwellenwert. Wie bereits erwähnt, hängt die Dämpfung gemäß dieser Ausgestaltung außerdem vom relativen Zeitpunkt $\tau$ während eines Referenz-Herzschlag-Zeitraum $H\_Zr_{ref}$ ab. Auf diese Weise lässt die Dämpfung sich an die aktuelle Herzaktivität des Patienten P anpassen, auch bei Unregelmäßigkeiten in der Herzaktivität.

In einer Ausgestaltung wird aus dem durchschnittlichen Leistungs-Signalabschnitt $Pow_{com,av}(i)$ ein Referenz-Dämpfungs-Signalabschnitt $Mod(i)$ erzeugt, beispielsweise gemäß der folgenden Rechenvorschrift:

$$Mod(i)(\tau) = min \{ Avg(i) / Pow_{com,av}(i)(\tau), 1\},$$

falls $\tau$ im Referenz-Herzschlag-Zeitraum $H\_Zr_{ref}$ liegt und $Pow_{com,av}(i)(\tau) > \varphi(i)$ gilt, und

$$Mod(i)(\tau) = 1 \text{ ansonsten.}$$

Jeder Signalwert $Mod(i)(\tau)$ des Referenz-Dämpfungs-Signalabschnitts $Mod(i)$ ist eine Zahl zwischen 0 und 1 (einschließlich).

Die Ausgestaltung, dass der Signalwert $Mod(i)(\tau)$ außerhalb des Referenz-Herzschlag-Zeitraums $H\_Zr_{ref}$ auf 1 gesetzt wird, stellt sicher, dass der Referenz-Dämpfungs-Signalabschnitt $Mod(i)$ nur für den aktuellen Herzschlag eine Dämpfung bewirkt.

In einer Verallgemeinerung wird jeder Wert für $Mod(i)$ gemäß der Rechenvorschrift

$$Mod(i) (\tau) = min \{F[Pow_{com,av}(i) (\tau)], 1\}$$

berechnet, wobei $F = F(u)$ eine in u fallende Funktion ist [je größer u, desto kleiner $F(u)$] und einen Wertebereich von 0 bis y hat und wobei y größer oder gleich 1 ist.

Wie in Figur 8 dargestellt ist, gibt es Alternativen zu der Ausgestaltung, die Dämpfung durch eine Multiplikation zu erzielen. In mehreren Ausgestaltungen, die in Figur 8 b) bis Figur 8 d) gezeigt werden, wird ein Schwellenwert $\alpha_X = \alpha_X(\tau)$ verwendet. In einer Ausgestaltung, die in Figur 8 d) gezeigt wird, werden zusätzlich zwei weitere Schwellenwerte $\beta_X = \beta_X(\tau)$ und $\beta_Y = \beta_Y(\tau)$ verwendet.

Figur 12 zeigt eine erfindungsgemäße Erweiterung des Funktionsschaltbilds von Figur 7. Gleiche Bezugszeichen haben die gleichen Bedeutungen wie in Figur 7.

Figur 12 zeigt schematisch einen Messwert-Aufbereiter 19. Dieser Messwert-Aufbereiter 19 bereitet das Roh-Signal $Sig_{raw}$ auf, welches von den Sensoren 2.1.1 bis 2.2.2 nach einer Signalverstärkung geliefert wird. Der Messwert-Aufbereiter 19 beseitigt rechnerisch niederfrequente Schwingungen, normiert das Roh-Signal $Sig_{raw}$ und liefert das Summen-Signal $Sig_{Sum}$.

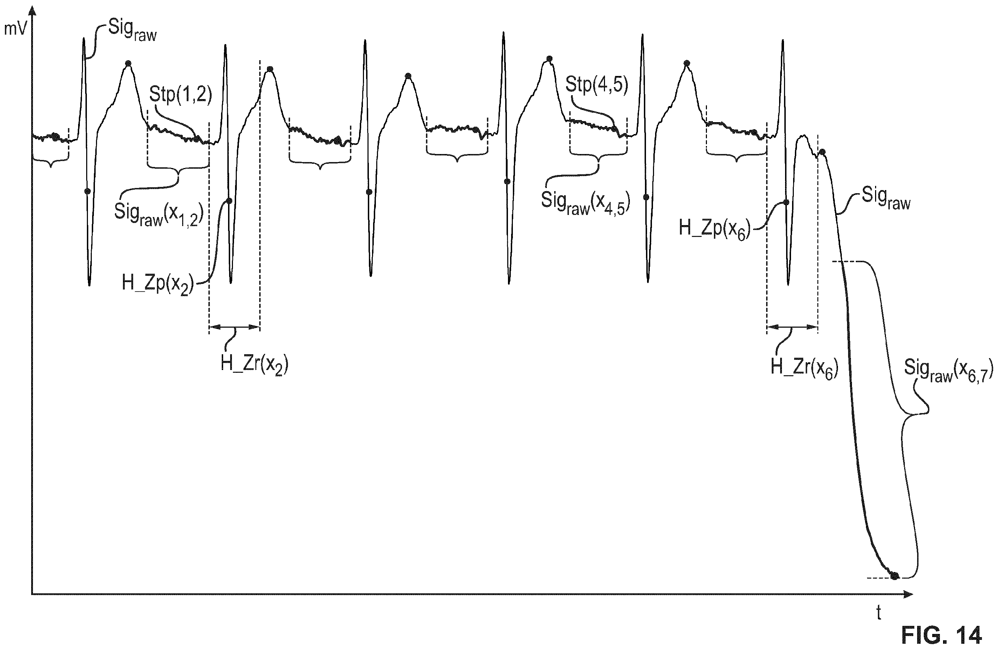

In einer Ausgestaltung subtrahiert der Messwert-Aufbereiter 19 vom Rohsignal $Sig_{raw}$ eine Art Durchschnittskurve (Baseline) BL. Figur 14 veranschaulicht eine bevorzugte Ausgestaltung, um die Durchschnittskurve (Baseline) BL zu berechnen. Dargestellt ist ein Segment des Roh-Signals $Sig_{raw}$, welches die sechs Herzschläge $x_1$, ..., $x_6$ umfasst. Eine Abfolge von Abschnitten $Sig_{raw}(x_{n,n+1})$, $Sig_{raw}(x_{n+1,n+2})$, ... des Roh-Signals $Sig_{raw}$ zwischen jeweils zwei aufeinanderfolgenden Herzschlag Zeiträumen $H\_Zr(x_n)$ und $H\_Zr(x_{n+1})$, $H\_Zr(x_{n+1})$ und $H\_Zr(x_{n+2})$, wird ermittelt. Der Abschnitt $Sig_{raw}(x_{1,2})$ liegt also zwischen den beiden Herzschlag-Zeiträumen $H\_Zr(x_1)$ und $H\_Zr(x_2)$ usw. Bevorzugt tritt zwischen dem Zeitraum, den der Abschnitt $Sig_{raw}(x_{n,n+1})$ überdeckt, und den beiden benachbarten Herzschlag-Zeiträumen $H\_Zr(x_n)$ und $H\_Zr(x_{n+1})$ ein vorgegebener zeitlicher Abstand von jeweils $\Delta t$ auf.

Für jeden Abschnitt $Sig_{raw}(x_{1,2})$, $Sig_{raw}(x_{2,3})$ wird jeweils ein Stützpunkt Stp(1,2), Stp(2,3), ... ermittelt. Stp(k,k+1) bezeichnet den Stützpunkt für den Abschnitt $Sig_{raw}(x_{k,k+1})$ (k=1,2,...). Durch diese Abfolge von Stützpunkten Stp(1,2), Stp(2,3), ... wird ein Spline gezogen. Der Abschnitt des Splines zwischen zwei benachbarten Stützpunkten ist ein Polynom. Bevorzugt wird als Spline ein Piecewise Cubic Hermite Interpolating Polynomial verwendet, wobei zwischen zwei benachbarten Stützpunkten ein Polynom dritter Ordnung auftritt.

Die nachfolgend beschriebenen Gruppen von Qualitätsmaßen (Qualitätsbewertungen, Qualitätsindikatoren) lassen sich unterscheiden. Diese Gruppen führen zu Qualitätsmaße Q[30], Q[31], Q[32], Q[33]. Diese Qualitätsmaße werden in der Initialisierungsphase und / oder in der Nutzphase berechnet und angewendet, was weiter unten detaillierter beschrieben wird.

Q[30]: Wie gut ist das Summen-Signal $Sig_{Sum}$, das aus den Messwerten der Sensoren erzeugt wurde? Mögliche Fehlerquellen sind:

- Die Messwerte der Sensoren am oder im Körper des Patienten P sind fehlerhaft, beispielsweise aufgrund eines schlechten oder gar fehlenden Kontakts einer Messelektrode mit dem Körper des Patienten P, was zu einem falschen Elektrodenwiderstand führt.
- Die Messwerte der Sensoren werden von Störgrößen überlagert, beispielsweise von Interferenzen von einem stationären Spannungsversorgungsnetz, elektrogalvanischen Störungen (Messelektrode wurde angefasst), anderen elektromagnetischen Störungen aus elektrischen oder elektronischen Geräten in der Nähe.
- Der Vorgang, die Messwerte der Sensoren an die Signalverarbeitungseinheit 5 zu übermitteln, ist einer Störung unterworfen, beispielsweise einem Kabelbruch oder einem Verbindungsabriss.
- Am Messwert-Aufbereiter 19 tritt eine technische Störung auf, beispielsweise eine Bereichsüberschreitung (out of Range) bei einem A-D-Wandler, eine leere Batterie, ein interner Kommunikationsabbruch.

In das Qualitätsmaß Q[30] fließt außerdem ein, wie gut die Baseline BL rechnerisch entfernt wurde.

Q[31]: Mit welcher Zuverlässigkeit wurden der Herzschlag-Zeitraum und / oder der Herzschlag-Zeitpunkt eines Herzschlags im Summen-Signal $Sig_{Sum}$ detektiert? Mögliche Einflussfaktoren auf das Qualitätsmaß Q[31] sind:

- Wie regelmäßig ist der Herzschlag?
- Sind die Verläufe der einzelnen Herzschläge ausreichend ähnlich, vgl. Figur 1?
- Sind die Signalabschnitte für die einzelnen Herzschläge hinreichend valide gefunden worden?

Q[32]: Wie zuverlässig ist ein Referenz-Dämpfungs-Signalabschnitt Mod(1), ..., Mod(n) oder ein angepasster Dämpfungs-Signalabschnitt Mod(1)(x), ..., Mod(n)(x) geeignet, um den Beitrag des kardiogenen Signals $Sig_{kar}$ zum Zwischen-Signal $Sig_{com}$ rechnerisch zu kompensieren? Diese Zuverlässigkeit kann sich auf einen einzelnen Herzschlag-Zeitraum beziehen, also von Herzschlag zu Herzschlag variieren, oder sich auf den Referenz-Herzschlag-Zeitraum $H\_Zr_{ref}$ beziehen (bevorzugt). Ein Dämpfungs-Signalabschnitt wird in einer Ausgestaltung aus einem Leistungs-Signalabschnitt hergeleitet. Eine Ausgestaltung, die Zuverlässigkeit Q[32] herzuleiten, ist daher die folgende: Sind ein Leistungs-Signalabschnitt $Pow_{com,i}(x)$ für einen Herzschlag x oder ein durchschnittlicher Leistungs-Signalabschnitt $Pow_{com,av}(i)$ plausibel, passen also zu Erwartungen an einen Leistungs-Signalabschnitt eines einzelnen Herzschlags und / oder zu den Erwartungen an einen durchschnittlichen Leistungs-Signalabschnitt $Pow_{com,av}(i)$? Anders formuliert: Wie gut beschreiben der Leistungs-Signalabschnitt $Pow_{com}(i)(x)$ oder der durchschnittliche Leistungs-Signalabschnitt $Pow_{com,av}(i)$ den Beitrag des kardiogenen Signals $Sig_{kar}$ zum Zwischen-Signal $Sig_{com}$ in einem Herzschlag-Zeitraum $H\_Zr(x)$?

In einer Ausgestaltung wird ein Qualitätsmaß Q[32] für einen Herzschlag x als Leistungs-Qualitätsmaß berechnet und fungiert als Gewichtsfaktor bei dem Vorgang, ein gewichtetes Mittel über mehrere Leistungsmaß-Stichprobenelemente $Pow_{com}(1)$, ..., $Pow_{com}(n)$ zu berechnen, um einen durchschnittlichen Leistungs-Signalabschnitt $Pow_{com,av}(i)$ herzuleiten. In einer Ausgestaltung fungiert das Qualitätsmaß Q[32] für einen durchschnittlichen Leistungs-Signalabschnitt $Pow_{com,av}(i)$ als Maß für die Zuverlässigkeit, mit der ein Referenz-Dämpfungs-Signalabschnitt Mod(i) für ein Frequenzband (Level) i den Beitrag des kardiogenen Signals $Sig_{res}$ zum Zwischen-Signalabschnitt $SigA_{com}(x)$, $SigA_{com}(y)$ für einen Herzschlag-Zeitraum $H\_Zr(x)$, $H\_Zr(y)$ oder für den Referenz-Herzschlag-Zeitraum $H\_Zr_{ref}$ beschreibt.

Q[33]: Passt der Zwischen-Signalabschnitt $SigA_{com}(x)$ für einen Herzschlag x zu vorgegebenen Erwartungen an einen Zwischen-Signalabschnitt ? Der Zwischen-Signalabschnitt $SigA_{com}(x)$ wurde abhängig von einem kardiogenen Signalabschnitt $SigA_{kar,ref}$ oder $SigA_{kar}(x)$ erzeugt. Eine Ausgestaltung ist daher die folgende: Passt der kardiogene Referenz-Signalabschnitt $SigA_{kar,ref}$, der für jeden Herzschlag verwendet wird, oder der für einen Herzschlag x angepasste kardiogene Signalabschnitt $SigA_{kar}(x)$ zu vorgegebenen Erwartungen an einen kardiogenen Signalabschnitt? Insbesondere: Hat der angepasste kardiogene Signalabschnitt $SigA_{kar}(x)$ einen zeitlichen Verlauf von Q bis T wie in Figur 1 gezeigt?

In Figur 7 und Figur 12 werden drei zusätzliche Funktionsblöcke gezeigt, welche diese Qualitätsmaße Q[30], Q[31], Q[32], Q[33] berechnen:

- Ein Funktionsblock 130 bewertet die Güte, mit der das Summen-Signal $Sig_{Sum}$ generiert wurde. Der Funktionsblock 130 liefert das Qualitätsmaß Q[30], das für jeden Herzschlag x jeweils einen Wert umfasst.
- Ein Funktionsblock 131 liefert das Qualitätsmaß Q[31], das für jeden Herzschlag x jeweils einen Wert umfasst.
- Ein Funktionsblock 132 liefert die Qualitätsmaße Q[32] und Q[33].
- In einer Ausgestaltung umfasst das jeweilige Qualitätsmaß Q[32] für jeden Herzschlag x jeweils einen einzigen Wert, in einer anderen Ausgestaltung einen zeitlichen Verlauf von Werten. Das Entsprechende gilt für das Qualitätsmaß Q [33].

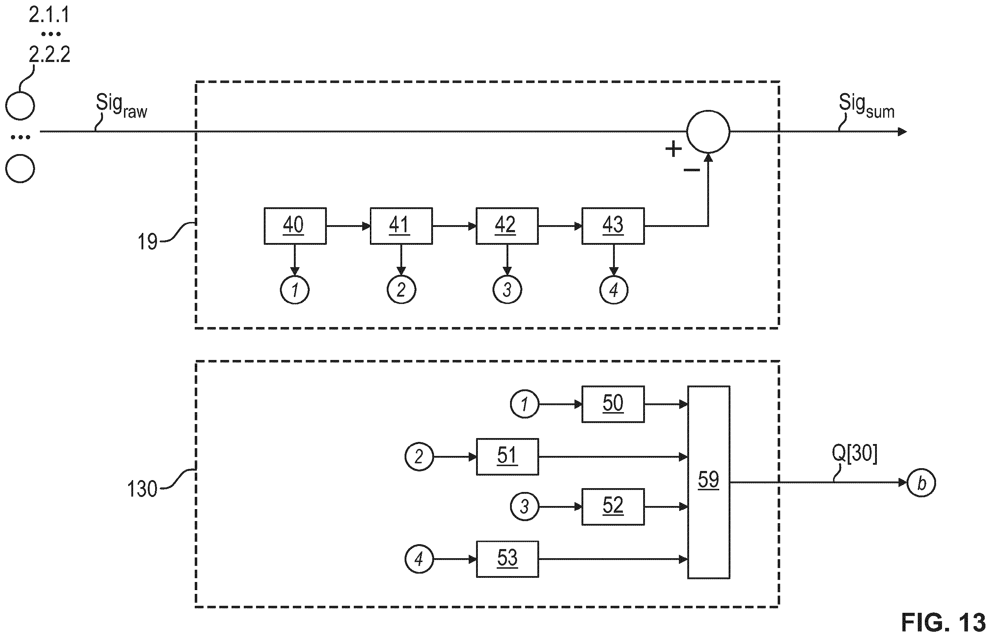

Figur 13 zeigt beispielhaft mehrere Funktionseinheiten des Messwert-Aufbereiters 19 sowie den Funktionsblock 130, der die Güte Q[30] bewertet, mit welcher der Messwert-Aufbereiter 19 aus dem Roh-Signal $Sig_{raw}$ das Summen-Signal $Sig_{Sum}$ generiert.

Möglich ist, dass die Herzaktivität des Patienten P auf mindestens zwei verschiedene Summen-Signale wirkt, insbesondere auf das jeweilige Summen-Signale von unterschiedlichen Sensoren. In einer Ausgestaltung werden unterschiedliche Herzschlag-Zeitpunkte detektiert. Sie stammen aber alle von demselben Herz und sind daher unterschiedliche Schätzungen für dasselbe Ereignis. In einer Ausgestaltung wird ein Herzschlag-Zeitpunkt H_Zp(x), H_Zp(y) aus einem Signal ausgewählt. In einer Ausgestaltung bewertet der Funktionsblock 131, wie weit die Schätzungen für einen Herzschlag-Zeitpunkt H_Zp(x), H_Zp(y) voneinander differieren, und berechnet das Qualitätsmaß Q[31] abhängig von den Unterschieden.

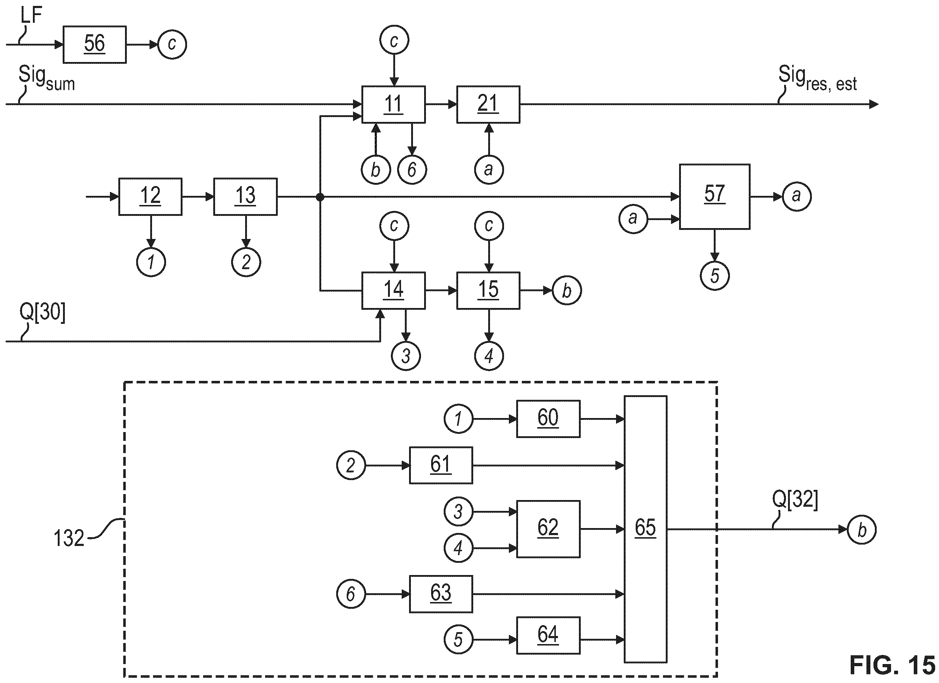

Figur 15 zeigt im Detail den Funktionsblock 132. Wie bereits dargelegt, bewertet der Funktionsblock 132, ob der ermittelte kardiogene Referenz-Signalabschnitt $SigA_{kar,ref}$ oder der angepasste kardiogene Signalabschnitt $SigA_{kar}(x)$ für einen Herzschlag x zu vorgegebenen Erwartungen an einen kardiogenen Signalabschnitt passen. Der Funktionsblock 132 liefert ein Qualitätsmaß Q[33]. Außerdem berechnet der Funktionsblock 132 das Qualitätsmaß Q[32].

Folgende Funktionseinheiten werden in Figur 15 gezeigt:

- Wie bereits erwähnt, identifiziert die Funktionseinheit 12 im Summen-Signal $Sig_{Sum}$ den jeweiligen Herzschlag-Zeitraum H_Zr(x) jedes Herzschlags x, bevorzugt die QRS-Phase.
- Die Funktionseinheit 13 ermittelt den jeweiligen charakteristischen Herzschlag-Zeitpunkt H_Zp(x) jedes Herzschlags x.
- Wie bereits erwähnt, überlagert die Funktionseinheit 14 rechnerisch die zeitrichtig positionierten N Summen-Signalabschnitte $SigA_{Sum}(x_1)$, ..., $SigA_{Sum}(x_N)$ für die letzten N Herzschläge $x_1$, ..., $x_N$.
- Die Funktionseinheit 15 erzeugt aus der Überlagerung von N Summen-Signalabschnitten $SigA_{Sum}(x_1)$, ..., $SigA_{Sum}(x_N)$ einen kardiogenen Referenz-Signalabschnitt $SigA_{kar,ref}$.
- Eine optionale Funktionseinheit 56 berechnet abhängig von einem Wert eines anthropologischen Parameters des Patienten P während des Herzschlags x einen formverändernden Faktor für den kardiogenen Referenz-Signalabschnitt $SigA_{kar,ref}$ und erzeugt dadurch für einen Herzschlag x einen angepassten kardiogenen Signalabschnitt $SigA_{kar}(x)$. Der anthropologischen Parameter ist beispielsweise der aktuelle Lungen-Füllstand oder die aktuelle Position des Patienten P. Eine beispielhafte Wirkungsweise dieser Funktionseinheit wird in DE 10 2019 006 866 A1 und US 2022 / 0 330 837 A1 beschrieben.
- Die Funktionseinheit 11 subtrahiert vom Summen-Signal $Sig_{Sum}$ den kardiogenen Referenz-Signalabschnitt $SigA_{kar,ref}$ oder einen angepassten kardiogenen Signalabschnitt $SigA_{kar}(x)$.
- Der optionale Dämpfungs-Funktionsblock 21 führt eine Dämpfung des kardiogenen Referenz-Signalabschnitts $SigA_{kar,ref}$ oder des angepassten kardiogenen Signalabschnitts $SigA_{kar}(x)$ durch, um verbleibende Anteile des kardiogenen Signals $Sig_{kar}$ zu entfernen.
- Die Funktionseinheit 57 führt eine Restleistungsanalyse durch und tauscht hierfür Signale mit dem Dämpfungs-Funktionsblock 21 aus. Bei einer solchen Restleistungsanalyse werden die Signalstärken von Dämpfungs-Signalabschnitten Mod(i) untersucht, und optional wird eine Dämpfung durchgeführt. Die Dämpfung wurde beispielhaft mit Bezug auf Figur 7 bis Figur 11 beschrieben.

Die Funktionseinheiten 12, 13, 16 und 21 führen die jeweiligen Rechenschritte mit einer hohen Abtast-Frequenz von wenigen Millisekunden vor, sodass das jeweilige Ergebnis bereits während des jeweiligen Herzschlags vorliegt. Die Funktionseinheiten 14, 15 und 57 führen die Rechenschritte mit einer niedrigeren, z.B. der oben erwähnten niedrigen, Abtast-Frequenz durch und verarbeiten die N Summen-Signalabschnitte $SigA_{Sum}(x_1)$, ..., $SigA_{Sum}(x_N)$ von N bereits abgeschlossenen Herzschlägen.

Wie bereits weiter oben erwähnt, wird ein durchschnittlicher Leistungs-Signalabschnitt $Pow_{com,av}(i)$ berechnet, vgl. Figur 12. Dieser durchschnittliche Leistungs-Signalabschnitt $Pow_{com,av}(i)$ beschreibt für das Level Nr. i den zeitlichen Verlauf einer elektrischen Leistung, wobei der zeitliche Verlauf einen einzigen relativen Herzschlag-Zeitraum T abdeckt. Der durchschnittliche Leistungs-Signalabschnitt $Pow_{com,av}(i)$ wird als ein gewichtetes Mittel über die M Signalanteil-Abschnitte $SigA_{com}(i)$ von M Herzschlägen berechnet. Ein Funktionsblock 101 positioniert die M Signalanteil-Abschnitte

$SigA_{com}(i)$ zeitrichtig zueinander bezüglich des Referenz-Herzschlag-Zeitraums $H\_Zr_{ref}$. Die Funktionseinheit 24 erzeugt aus den zeitrichtig positionierten M Signalanteil-Abschnitte $SigA_{com}(i)$ den durchschnittlichen Leistungs-Signalabschnitt $Pow_{com,av}(i)$ und hieraus den Referenz-Dämpfungs-Signalabschnitt Mod(i), so wie dies weiter oben beschrieben wurde.

Die Funktionseinheit 24 von Figur 7 und Figur 12 verwendet jeweils mindestens eines der vier Qualitätsmaße Q [30] bis Q[34] für folgende Aufgaben:

- in der Initialisierungsphase für die Aufgabe, die Gewichtsfaktoren zu berechnen, mit deren Hilfe für jedes Level Nr. i der jeweilige durchschnittliche Leistungs-Signalabschnitt $Pow_{com,av}(i)$ berechnet wird,
- ebenfalls in der Initialisierungsphase für die Aufgabe, den Referenz-Dämpfungs-Signalabschnitt Mod(i) zu erzeugen,
- in der Nutzphase für die optionale Aufgabe, den Referenz-Dämpfungs-Signalabschnitt Mod(i) zu aktualisieren, und
- in der Nutzphase für die Aufgabe, unter Verwendung des Referenz-Dämpfungs-Signalabschnitts Mod(i) den angepassten Dämpfungs-Signalabschnitt Mod(i)(x) für einen Herzschlag x zu berechnen.

Für jede dieser Aufgaben berechnet die Funktionseinheit 24 jeweils ein Gesamt-Qualitätsmaß Q und verwendet für diese Berechnung mindestens ein Qualitätsmaß Q[30] bis Q[34], bevorzugt mehrere Qualitätsmaße. Eine Regel ist, dass das Gesamt-Qualitätsmaß Q umso schlechter ist, je geringer ein oben genannter und verwendeter Gewichtsfaktor , ist. Außerdem ist der angepasste Dämpfungs-Signalabschnitt Mod(i)(x) kleiner als oder höchstens genauso groß wie der Referenz-Dämpfungs-Signalabschnitt Mod(i) und umso kleiner, je schlechter das Gesamt-Qualitätsmaß Q ist.

In der Nutzphase verwendet die Signalverarbeitungseinheit 5 für einen Herzschlag x und für ein Level Nr. i den zuvor ermittelten Referenz-Dämpfungs-Signalabschnitt Mod(i), um einen angepassten Dämpfungs-Signalabschnitt Mod(i)(x) zu erzeugen. Eine Wirkung dieser Dämpfung ist die folgende: In der Nutzphase wird die Dämpfung verstärkt, falls und solange ein schlechtes Gesamt-Qualitätsmaß Q ermittelt wurde. Ein Signalabschnitt des Signals $Sig_{com}$ mit einer schlechten Qualität Q wird daher im Vergleich zu anderen Signalabschnitten stärker gedämpft.

Insbesondere sind folgende Realisierungsformen möglich, wie der angepasste Dämpfungs-Signalabschnitt Mod(i)(x) in der Nutzphase abhängig vom Gesamt-Qualitätsmaß Q verändert wird:

- Jeder Wert des angepasste Dämpfungs-Signalabschnitts Mod(i)(x) wird mit einem Faktor $\alpha < 1$ multipliziert, wobei dieser Faktor $\alpha < 1$ aus dem Qualitätsmaß Q[30], Q[31], Q[32], Q[33] hergeleitet wird und umso kleiner ist, je schlechter das Gesamt-Qualitätsmaß Q ist.
- Nur diejenigen Werte des angepassten Dämpfungs-Signalabschnitts Mod(i)(x) werden mit dem Faktor $\alpha < 1$ multipliziert, die kleiner oder gleich einer vorgegebenen oberen Schranke $\beta$ sind, wobei gilt: $0 < \beta < 1$.
- Jeder Wert des angepassten Dämpfungs-Signalabschnitts Mod(i)(x) wird um einen festen Wert $\Delta >= 0$ verringert, wobei der feste Wert $\Delta$ umso größer ist, je schlechter das Gesamt-Qualitätsmaß Q ist. Der Wert des angepassten Dämpfungs-Signalabschnitts Mod(i)(x) wird aber höchstens auf 0 reduziert, es treten also keine negativen Werte auf.
- Nur diejenigen Werte des angepassten Dämpfungs-Signalabschnitts Mod(i)(x) werden um den festen Wert $\Delta$ reduziert, die kleiner oder gleich der oben genannten oberen Schranke $\beta$ sind.
- Jeder Wert des angepassten Dämpfungs-Signalabschnitts Mod(i)(x) wird mit dem Faktor $\alpha$ multipliziert oder um den festen Wert $\Delta$ reduziert, je nachdem welches Vorgehen zum kleineren Ergebnis führt. Wiederum werden Werte kleiner Null vermieden.

Zusätzlich oder anstelle der gerade genannten Realisierungsformen wird der angepasste Dämpfungs-Signalabschnitt Mod(i)(x) rechnerisch geglättet, insbesondere durch Anwendung eines gleitenden Durchschnitts (moving average filtering).

Der gerade genannte Faktor $\alpha$ und / oder der gerade genannte feste Wert $\Delta$ kann für jedes Level i, also für jedes Frequenzband, dasselbe sein. Möglich ist auch, dass in der Nutzphase für jedes Level i (i = 1, ..., n) jeweils bis zu drei einzelne Qualitätsmaße Q[30](i), Q[31](i), Q[32](i) ermittelt werden und hieraus ein eigenes Gesamt-Qualitätsmaß Q = Q(i) hergeleitet wird. Entsprechend werden für jedes Level i ein eigener Faktor $\alpha(i)$ und / oder ein eigener fester Wert $\Delta(i)$ hergeleitet und so wie gerade beschrieben verwendet.

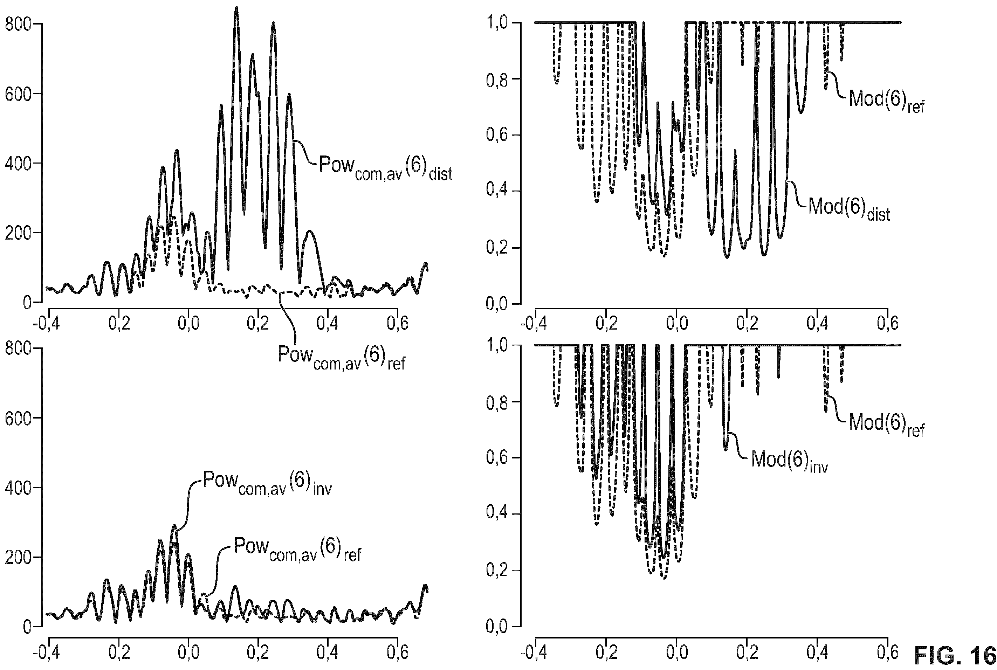

Figur 16 veranschaulicht eine Verbesserung, welche durch die Erfindung erzielt wird. In der linken Spalte sind beispielhaft zeitliche Verläufe des gemittelten Leistungs-Signalabschnitts $Pow_{com,av}(6)$ für das Level 6 dargestellt, in der rechten Spalte zeitliche Verläufe des resultierenden Referenz-Dämpfungs-Signalabschnitts Mod(6). Die obere Zeile veranschaulicht ein Ergebnis eines Verfahrens, welches die Erfindung nicht nutzt, die untere Zeile das Ergebnis des erfindungsgemäßen Verfahrens. In jedem Diagramm sind jeweils ein zeitlicher Verlauf bei einer Situation frei von Störungen und ein Verlauf beim Vorliegen von Störungen dargestellt. Im Einzelnen bedeuten

$Pow_{com,av}(6)_{dist}$   gemittelter Leistungs-Signalabschnitt beim Vorliegen von Störungen, gemäß dem Stand der Technik erzielt,

(fortgesetzt)

| | |
|---|---|
| Pow$_{com,av}(6)_{inv}$ | gemittelter Leistungs-Signalabschnitt beim Vorliegen von Störungen, erfindungsgemäß erzielt, |
| Pow$_{com,av}(6)_{ref}$ | gemittelter Leistungs-Signalabschnitt ohne Störungen, |
| Mod$(6)_{dist}$ | Referenz-Dämpfungs-Signalabschnitt beim Vorliegen von Störungen, gemäß dem Stand der Technik erzielt, |
| Mod$(6)_{inv}$ | Referenz-Dämpfungs-Signalabschnitt beim Vorliegen von Störungen, erfindungsgemäß erzielt, |
| Mod$(6)_{ref}$ | Referenz-Dämpfungs-Signalabschnitt ohne Störungen. |

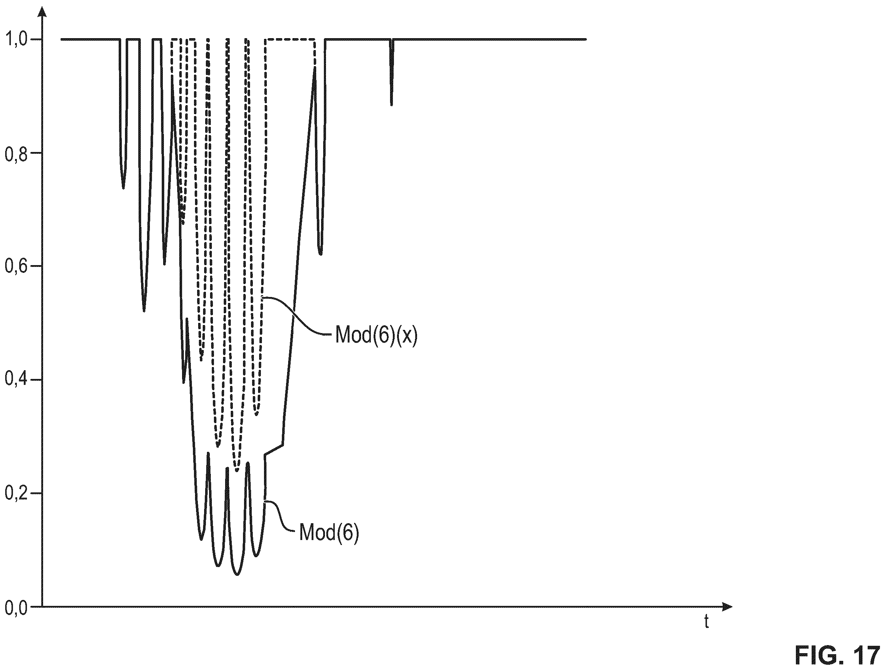

Figur 17 veranschaulicht beispielhaft einen Referenz-Dämpfungs-Signalabschnitt Mod(6) und einen angepassten Dämpfungs-Signalabschnitt Mod(6)(x) für den Herzschlag x. Auf der x-Achse ist die Zeit t aufgetragen, auf der y-Achse der Signalwert, der zwischen 0 und 1 liegt.

**Bezugszeichenliste**

| 1 | Beatmungsgerät, beatmet künstlich und / oder überwacht den Patienten P, umfasst die Signalverarbeitungseinheit 5 |
|---|---|
| 2.1 | interkostales (herznahes) von Messelektroden auf der Haut des Patienten P, liefert Messwerte für das elektrische Summen-Signal Sig$_{Sum}$ |
| 2.1.1, 2.1.2, | Messelektroden des interkostalen Paars 2.1 |
| 2.2 | zwerchfellnahes Paar von Messelektroden auf der Haut des Patienten P, liefert weitere Messwerte für das Summen-Signal Sig$_{Sum}$ |
| 2.2.1, 2.2.2 | Messelektroden des zwerchfellnahen Paares 2.2 |
| 3 | pneumatischer Sensor vor dem Mund des Patienten P, misst den Volumenfluss Vol' und den Atemwegsdruck P$_{aw}$ |
| 4 | optischer Sensor mit einem Bildaufnahmegerät und einer Bildverarbeitungseinheit, misst die Geometrie des Körpers des Patienten P, aus welcher rechnerisch der aktuelle Lungen-Füllstand Vol hergeleitet wird |
| 5 | Signalverarbeitungseinheit, führt die Schritte des erfindungsgemäßen Verfahrens durch, hat Lesezugriff und Schreibzugriff auf den Datenspeicher 9 |
| 6 | Sonde in der Speiseröhre Sp, misst den pneumatischen Druck P$_{es}$ in der Speiseröhre Sp |
| 7 | Manschette um ein Handgelenk des Patienten P, hält den Katheter 17, welcher invasiv den zeitlichen Verlauf des Blutdrucks misst |
| 8.1 | Sensor in Form eines Finger-Clips an einem Finger des Patienten P, misst nicht-invasiv den Grad der Sättigung des Bluts mit Sauerstoff |
| 8.2 | Sensor in Form eines Finger-Clips an einem weiteren Finger des Patienten P, misst nicht-invasiv den Blutdruck des Patienten P |
| 9 | Datenspeicher, auf den die Signalverarbeitungseinheit 5 wenigstens zeitweise Lesezugriff und Schreibzugriff hat und in dem der kardiogene Referenz-Signalabschnitt SigA$_{kar,ref}$ und die respiratorischen Referenz-Dämpfungs-Signalabschnitte Mod(i) abgespeichert sind |
| 10 | Funktionseinheit des Kompensations-Funktionsblocks 20: generiert das synthetische kardiogene Signal Sig$_{kar,syn}$ |
| 11 | Funktionseinheit des Kompensations-Funktionsblocks 20: kompensiert unter Verwendung des synthetischen kardiogenen Signals Sig$_{kar,syn}$ den Einfluss des kardiogenen Signals Sig$_{kar}$ auf das Summen-Signal Sig$_{Sum}$, beispielsweise durch Subtraktion von Sig$_{kar,syn}$ |
| 12 | Funktionseinheit der Signalverarbeitungseinheit 5: erkennt im Summen-Signal Sig$_{Sum}$ die jeweilige QRS-Zeitspanne (QRS-Segment) jedes Herzschlags |
| 13 | Funktionseinheit der Signalverarbeitungseinheit 5: detektiert den genauen Herzschlag-Zeitpunkt H_Zp(n) jedes Herzschlags |

(fortgesetzt)

| | |
|---|---|
| 14 | Funktionseinheit des Kompensations-Funktionsblocks 20: überlagert rechnerisch N Summen-Signalabschnitte $SigA_{Sum}(x_1)$, ..., $SigA_{Sum}(x_N)$ für die letzten N Herzschläge |
| 15 | Funktionseinheit des Kompensations-Funktionsblocks 20: erzeugt einen kardiogenen Referenz-Signalabschnitt $SigA_{kar,ref}$ |
| 16 | Funktionseinheit des Kompensations-Funktionsblocks 20: positioniert die kardiogenen Referenz-Signalabschnitte $SigA_{kar,ref}$ oder die angepassten kardiogenen Signalabschnitte $SigA_{kar}(x)$ abhängig vom Herzschlag-Zeitpunkt $H\_Zp(x)$ zeitrichtig, setzt die positionierten kardiogenen Referenz-Signalabschnitte $SigA_{kar,ref}$ zu dem synthetischen kardiogenen Signal $Sig_{kar,syn}$ zusammen |
| 17 | Katheter, von der Manschette 7 gehalten, misst invasiv den zeitlichen Verlauf des Blutdrucks des Patienten P |
| 19 | Messwert-Aufbereiter, erzeugt aus den Messwerten der Messelektroden 2.1.1 bis 2.2.2 das Summen-Signal $Sig_{Sum}$ |
| 20 | Kompensations-Funktionsblock: erzeugt das synthetische kardiogene Signal $Sig_{kar,syn}$ und das Kompensations-Signal $Sig_{com}$ |
| 21 | Dämpfungs-Funktionsblock: erzeugt durch Dämpfung aus dem Kompensations-Signal $Sig_{com}$ das geschätzte respiratorische Signal $Sig_{res,est}$ |
| 22 | Funktionseinheit des Dämpfungs-Funktionsblocks 21: zerlegt das Kompensations-Signal $Sig_{com}$ in n Signalanteil-Abschnitte $SigA_{com}(1)(x)$, ..., $SigA_{com}(n)(x)$ für n Level |
| 23 | Funktionseinheit des Dämpfungs-Funktionsblocks 21: erzeugt aus dem Kompensations-Signal $Sig_{com}$ durch Dämpfung das geschätzte respiratorische Signal $Sig_{res,est}$ |
| 23(i) | Funktionseinheit des Dämpfungs-Funktionsblocks 21: erzeugt aus dem Signalanteil-Abschnitt $SigA_{com}(i)$ den gedämpften Signalanteil-Abschnitt $SigA_{com,d}(i)$ (i = 1, ..., n) |
| 24 | Funktionseinheit des Dämpfungs-Funktionsblocks 21: erzeugt den Referenz-Dämpfungs-Signalabschnitt Mod(i) (i = 1,..., n) |
| 25 | Funktionseinheit des Dämpfungs-Funktionsblocks 21: setzt die gedämpften Signalanteil-Abschnitte $SigA_{com,d}(1)$, ... , $SigA_{com,d}(n)$ durch Rücktransformation zum neuesten Abschnitt $SigA_{com,d}$ des gedämpften kompensierten Signals $SigA_{com,d}$ zusammen, wobei dieser Abschnitt als neuester Abschnitt des geschätzten respiratorischen Signal $Sig_{res,est}$ verwendet wird |
| 26 | Funktionseinheit in der Funktionseinheit 23 / 23(i): wendet das Referenz-Dämpfungs-Signal Mod(i) auf den Signalanteil $SigA_{com}(i)(x)$ an und erzeugt den gedämpften Signalanteil $SigA_{com,d}(i)(x)$ (i = 1, ..., n) |
| 30 | Funktionseinheit des Dämpfungs-Funktionsblocks 21: erzeugt aus dem Kompensations-Signal $Sig_{com}$ unter Verwendung der charakteristischen Herzschlag-Zeitpunkte die Kompensations-Signalabschnitte |
| 32 | optionale Funktionseinheit: verzögert das Summen-Signal $Sig_{Sum}$ für die Laufzeit, die zum Ermitteln des charakteristischen Herzschlag-Zeitpunkts $H\_Zp(x)$ erforderlich ist |
| 40 | Funktionseinheit: entdeckt in dem Roh-Signal $Sig_{raw}$ das QRS-Segment, |
| 41 | Funktionseinheit: detektiert die Länge eines aktuell ausgewerteten Abschnitts in dem Roh-Signal $Sig_{raw}$ |
| 42 | Funktionseinheit: detektiert einen Stützpunkt im aktuell ausgewerteten Abschnitt |
| 43 | Funktionseinheit: konstruiert durch Interpolation für jeden Herzschlag jeweils einen Spline |
| 50 | Funktionseinheit: bewertet die Regelmäßigkeit, mit der die Funktionseinheit 40 die QRS-Segmente detektiert |
| 51 | Funktionseinheit: detektiert Auswerte-Abschnitte, die besonders lang oder besonders kurz sind |
| 52 | Funktionseinheit: ermittelt die Standardabweichung der Zufallsvariable |

(fortgesetzt)

| | |
|---|---|
| 53 | Funktionseinheit: bewertet die Änderungen zwischen den Splines von zwei unmittelbar aufeinanderfolgenden Herzschlägen |
| 56 | Funktionseinheit: berechnet abhängig vom aktuellen Lungen-Füllstand einen formverändernden Faktor für den kardiogenen Referenz-Signalabschnitt $SigA_{kar,ref}$ und erzeugt dadurch für einen Herzschlag x einen kardiogener Signalabschnitt $SigA_{kar}(x)$ |
| 57 | optionale Funktionseinheit: analysiert die Restleistung |
| 59 | Funktionseinheit: berechnet aus den einzelnen Qualitätsmaßen der Funktionseinheiten 50 bis 53 das Qualitätsmaß Q[30] |
| 60 | Funktionseinheit: bewertet die Qualität, mit der die Funktionseinheit 13 den genauen Herzschlag-Zeitpunkt H_Zp(x) jedes Herzschlags x detektiert |
| 61 | Funktionseinheit: bewertet die Qualität, mit der ein kardiogener Referenz-Signalabschnitt $SigA_{kar,ref}$ oder ein angepasster kardiogener Signalabschnitt $SigA_{kar}(x)$ für den Herzschlag x generiert wird |
| 62 | Funktionseinheit: bewertet die Qualität, mit der die Funktionseinheit 16 vom Summen-Signal $Sig_{Sum}$ den kardiogenen Referenz-Signalabschnitt $SigA_{kar,ref}$ oder den angepassten kardiogener Signalabschnitt $SigA_{kar}(x)$ subtrahiert |
| 63 | Funktionseinheit: bewertet die Qualität, mit der die Funktionseinheit 57 die Restleistung analysiert hat |
| 64 | Funktionseinheit: berechnet das Qualitätsmaß Q[32] |
| 101 | Funktionsblock, positioniert die M Signalanteil-Abschnitte $SigA_{com}(i)$ zeitrichtig zueinander bezüglich des Referenz-Herzschlag-Zeitraums $H\_Zr_{ref}$ |
| 130 | Funktionsblock: bewertet die Güte, mit der aus dem Roh-Signal $Sig_{raw}$ das Summen-Signal $Sig_{Sum}$ generiert wurde, liefert das Qualitätsmaß Q[30], umfasst die Funktionseinheiten 50 bis 53 und 59 |
| 131 | Funktionsblock: bewertet, mit welcher Zuverlässigkeit durch Auswertung des Summen-Signals $Sig_{Sum}$ der charakteristische Herzschlag-Zeitpunkt H_Zp(x) detektiert wurde, liefert das Qualitätsmaß Q[31], umfasst die Funktionseinheiten |
| 132 | Funktionsblock: bewertet die Güte, mit der der kardiogene Referenz-Signalabschnitt $SigA_{kar,ref}$ oder der ermittelte kardiogene Signalabschnitt $SigA_{kar,syn}(x)$ für einen Herzschlag x ermittelt wurde, liefert das Qualitätsmaß Q[32], umfasst die Funktionseinheiten 60 bis 65 |
| Akt | optionale Aktualisierung in der Nutzphase des Referenz-Dämpfungs-Signalabschnitts Mod(i) |
| Atm(1), ... | Zeitspannen von Atemzügen |
| Avg(i) | mittlerer Signalwert für das Level Nr. i (i = 1, ..., n), aus dem gemittelten Leistungs-Signalabschnitt $Pow_{com,av}(i)$ berechnet (i = 1, ..., n) |
| H_Zp(x), H_Zp(y) | charakteristischer Herzschlag-Zeitpunkt des Herzschlags x bzw. y, von der Funktionseinheit 13 detektiert |
| H_Zr(x), H_Zr(y) | Herzschlag-Zeitraum des Herzschlags x bzw. y |
| $H\_Zr_{ref}$ | Referenz-Herzschlag-Zeitraum, vom kardiogenen Referenz-Signalabschnitt $SigA_{kar,ref}$ und vom Referenz-Dämpfungs-Signalabschnitt Mod(i) überdeckt |
| M | Anzahl der Herzschläge, die verwendet werden, um in der Initialisierungsphase den durchschnittliche Leistungs-Signalabschnitt $Pow_{com,av}(i)$ für das Level Nr. i zu berechnen |
| Mod(i) | Referenz-Dämpfungs-Signalabschnitt für das Level Nr. i, deckt den Referenz-Herzschlag-Zeitraum $H\_Zr_{ref}$ ab |
| Mod(i)(x) | angepasster Dämpfungs-Signalabschnitt für das Level Nr. i und den Herzschlag x, deckt den Herzschlag-Zeitraum Hab |
| n | Anzahl der Level (Frequenzbänder), in die das Kompensations-Signal $Sig_{com}$ zerlegt wird |

(fortgesetzt)

| | |
|---|---|
| N | Anzahl der Herzschläge, die in der Initialisierungsphase für die Generierung des kardiogenen Referenz-Signalabschnitts $SigA_{kar,ref}$ verwendet werden |
| P | Patient, wird mit Hilfe des Beatmungsgerätes 1 künstlich beatmet |
| $P_{aw}$ | Maß für den Atemwegsdruck (pressure in airway) |
| $P_{es}$ | Maß für den Speiseröhrendruck (pressure in esophagus) |
| $P_{mus}$ | pneumatisches Maß für die eigene Atmungsaktivität des Patienten P |
| $Pow_{com}(i)(x)$ | Leistungs-Signalabschnitt für das Level Nr. i (i=1,...,n) und für einen Herzschlag x |
| $Pow_{com,av}(i)$ | durchschnittlicher Leistungs-Signalabschnitt für das Level Nr. i, als gewichtetes Mittel von M einzelnen Leistungssignal-Abschnitten $Pow_{com}(i)(x)$ berechnet, deckt einen Referenz-Herzschlag-Zeitraum $H\_Zr_{ref}$ ab |
| $\varphi(i)$ | Schwellenwert (threshold) für das Level Nr. i (i = 1, ..., n) |
| Q[30] | Qualitätsmaß für die Güte, mit der aus den Messwerten der Sensoren 2.1.1 bis 2.2.2 das Summen-Signal $Sig_{Sum}$ generiert wurde, vom Funktionsblock 30 berechnet |
| Q[31] | Qualitätsmaß für die Zuverlässigkeit, mit der der charakteristische Herzschlag-Zeitpunkt $H\_Zp(x)$ detektiert wurde, vom Funktionsblock 31 berechnet |
| Q[32] | Qualitätsmaß für die Plausibilität eines durchschnittlichen Leistungs-Signalabschnitts $Pow_{com,av}(i)$, also wie gut er zu einem Herzschlag und / oder zu den Erwartungen an einen durchschnittlichen Leistungs-Signalabschnitt passt |
| Q[33] | Qualitätsmaß für die Güte, mit der der kardiogene Referenz-Signalabschnitt $SigA_{kar,ref}$ oder der ermittelte kardiogenen Signalabschnitt $SigA_{kar,syn}(x)$ für einen Herzschlag x ermittelt wurde, vom Funktionsblock 32 berechnet |
| Sigcom | Kompensations-Signal, wird vom Kompensations-Funktionsblock 20 durch Kompensation des Beitrags des synthetischen kardiogenen Signals $Sig_{kar,syn}$ zum Summen-Signal $Sig_{Sum}$ generiert, fungiert als Zwischen-Signal |
| $SigA_{com}(x)$ | Abschnitt des Kompensations-Signals $Sig_{com}$ für den Herzschlag x, fungiert als Zwischen-Signalabschnitt |
| $SigA_{com}(i)(x)$ | Signalanteil-Abschnitt für das Level Nr. i (i = 1, ..., n) des Abschnitts $SigA_{com}(x)$ für den Herzschlag x, von der Funktionseinheit 22 durch Zerlegung des Kompensations-Signals $Sig_{com}$ erzeugt |
| $Sig_{com,d}$ | gedämpftes Kompensations-Signal $Sig_{com}$ |
| $SigA_{com,d}(x)$ | gedämpfter Signalanteil für den Herzschlag x |
| $SigA_{com,d}(i)(x)$ | gedämpfter Signalanteil-Abschnitt für das Level Nr. i (i = 1, ..., n) und den Herzschlag x, von der Funktionseinheit 23(i) erzeugt |
| $Sig_{kar}$ | tatsächliches kardiogenes Signal, bewirkt die Herzaktivität des Patienten P, durch das synthetische kardiogene Signal $Sig_{kar,syn}$ geschätzt |
| $Sig_{kar,syn}$ | synthetisches kardiogenes Signal, ist eine Schätzung für das kardiogene Signal $Sig_{kar}$, von der Funktionseinheit 10 aus den Signalabschnitten $SigA_{kar,syn}(x)$ erzeugt |
| $SigA_{kar,syn}(x)$ | Abschnitt für den Herzschlag x des synthetischen kardiogenen Signals $Sig_{kar,syn}$ |
| $SigA_{kar,ref}$ | kardiogener Referenz-Signalabschnitt, beschreibt näherungsweise den Verlauf des kardiogenen Signals $Sig_{kar}$ während eines einzigen Herzschlags, bezieht sich auf den Referenz-Herzschlag-Zeitraum $H\_Zr_{ref}$ |
| $Sig_{raw}$ | Roh-Signal von den Messelektroden 2.1.1 bis 2.2.2 |
| $Sig_{raw}(X_{k,k+1})$ | Abschnitt des Roh-Signals $Sig_{raw}$ zwischen den beiden Herzschlag-Zeiträumen $H\_Zr(x_k)$ und $H\_Zr(x_{k+1})$ |
| $Sig_{res}$ | zu ermittelndes respiratorisches Signal, bewirkt die eigene Atmungsaktivität des Patienten P |
| $Sig_{res,est}$ | erfindungsgemäß ermittelte Schätzung für das zu ermittelnde respiratorische Signal $Sig_{res}$ |

(fortgesetzt)

| | |
|---|---|
| $Sig_{sum}$ | elektrisches Summen-Signal, von der Signalverarbeitungseinheit 5 erzeugt, umfasst eine Überlagerung des respiratorischen Signals $Sig_{res}$ mit dem kardiogenen Signal $Sig_{kar}$ |
| $SigA_{Sum}(x)$ | Abschnitt des Summen-Signals $Sig_{Sum}$ für den Herzschlag-Zeitraum H_Zr(x) des Herzschlags x |
| Stp(k,k+1) | Stützpunkt für den Abschnitt $Sig_{raw}(x_{k,k+1})$ |
| Sp | Speiseröhre des Patienten P |
| T | Zeit im Referenz-Herzschlag-Zeitraum $H\_Zr_{ref}$ |
| Vol' | Volumenfluss |
| Zw | Zwerchfell des Patienten P |

**Patentansprüche**

1.  Signalverarbeitungseinheit (5) zum Ermitteln einer Schätzung ($Sig_{res,est}$) für ein respiratorisches Signal ($Sig_{res}$),

    wobei das respiratorische Signal ($Sig_{res}$) mit der Ventilation der Lunge eines Patienten (P) korreliert und die Ventilation der Lunge durch eine eigene Atmungsaktivität und / oder durch eine künstliche Beatmung des Patienten (P) verursacht wird,
    wobei ein Referenz-Herzschlag-Zeitraum ($H\_Zr_{ref}$) und eine Nutzphase vorgegeben sind,
    wobei die Signalverarbeitungseinheit (5) dazu ausgestaltet ist, automatisch
    Messwerte von mindestens einem Summen-Signal-Sensor (2.1.1 bis 2.2.2) zu empfangen,
    wobei der oder jeder verwendete Summen-Signal-Sensor (2.1.1 bis 2.2.2) dazu ausgestaltet ist, ein im und / oder am Körper des Patienten (P) erzeugtes Signal zu messen,
    unter Verwendung von empfangenen Messwerten ein Summen-Signal ($Sig_{Sum}$) zu erzeugen,
    wobei das Summen-Signal ($Sig_{Sum}$) eine Überlagerung

    - des zu schätzenden respiratorischen Signals ($Sig_{res}$) und
    - eines kardiogenen Signals ($Sig_{kar}$), welches mit der Herzaktivität des Patienten (P) korreliert,

    umfasst,
    unter Verwendung des Summen-Signals ($Sig_{Sum}$)

    - mehrere Herzschläge und
    - für jeden detektierten Herzschlag jeweils einen charakteristischen Herzschlag-Zeitraum [H_Zr(x), H_Zr(y)], in dem dieser Herzschlag stattfindet,

    zu detektieren,
    ein Zwischen-Signal ($Sig_{com}$) zu berechnen und für die Berechnung des Zwischen-Signals ($Sig_{com}$) den Einfluss der Herzaktivität auf das Summen-Signal ($Sig_{Sum}$) wenigstens näherungsweise rechnerisch zu kompensieren,
    in einer ersten Alternative mindestens einen Referenz-Dämpfungs-Signalabschnitt [Mod(1), ..., Mod(n)] zu berechnen und in einer zweiten Alternative den oder jeden Referenz-Dämpfungs-Signalabschnitt [Mod(1), ..., Mod(n)] durch einen Lesezugriff auf einen Datenspeicher (9) zu ermitteln,
    wobei der oder jeder Referenz-Dämpfungs-Signalabschnitt [Mod(1), ..., Mod(n)] mit dem durchschnittlichen zeitlichen Verlauf des Beitrags des kardiogenen Signals ($Sig_{kar}$) zum Zwischen-Signal ($Sig_{com}$) im Referenz-Herzschlag-Zeitraum ($H\_Zr_{ref}$) korreliert,
    für jeden detektierten Herzschlag, der in die Nutzphase fällt,

    - jeweils einen Zwischen-Signalabschnitt [$SigA_{com}(x)$] als einen Abschnitt des Zwischen-Signals ($Sig_{com}$) zu erzeugen,
    wobei der Zwischen-Signalabschnitt [$SigA_{com}(x)$] im Herzschlag-Zeitraum [H_Zr(x), H_Zr(y)] dieses Herzschlags liegt, und
    - aus dem Zwischen-Signalabschnitt [$SigA_{com}(x)$] einen gedämpften Zwischen-Signalabschnitt [$SigA_{com,d}(x)$] für den Herzschlag-Zeitraum [H_Zr(x), H_Zr(y)] dieses Herzschlags zu erzeugen,

wobei der Einfluss des kardiogenen Signals (Sig_{kar}) auf den gedämpften Zwischen-Signalabschnitt [SigA_{com,d}(x)] kleiner als oder höchstens genauso groß wie der Einfluss des kardiogenen Signals (Sig_{kar}) auf den Zwischen-Signalabschnitt [SigA_{com}(x)] ist, und

die gedämpften Zwischen-Signalabschnitte [SigA_{com,d}(x)]
unter Verwendung der detektierten charakteristischen Herzschlag-Zeiträume [H_Zr(x), H_Zr(y)]
zur Schätzung (Sig_{res,est}) für das respiratorische Signal (Sig_{res}) zusammenzusetzen,
wobei die Signalverarbeitungseinheit (5) weiterhin dazu ausgestaltet ist, für jeden detektierten Herzschlag

- bei der ersten Alternative den Referenz-Dämpfungs-Signalabschnitt [Mod(1), ..., Mod(n)] und
- bei der zweiten Alternative einen angepassten Dämpfungs-Signalabschnitt [Mod(1)(x), ..., Mod(n)(x)]

auf den Zwischen-Signalabschnitt [SigA_{com}(x)] anzuwenden und durch die Anwendung den gedämpften Zwischen-Signalabschnitt [SigA_{com,d}(x)] für den Herzschlag zu erzeugen,
wobei die Signalverarbeitungseinheit (5) weiterhin dazu ausgestaltet ist,
mindestens eines der folgenden Qualitätsmaße (Q[30], Q[31], Q[32], Q[33]) zu berechnen:

- ein Qualitätsmaß (Q[30]) für die Zuverlässigkeit, mit der der oder jeder verwendete Summen-Signal-Sensor (2.1.1 bis 2.2.2) die jeweiligen Messwerte misst und / oder die Zuverlässigkeit, mit der die Signalverarbeitungseinheit (5) aus diesen Messwerten das Summen-Signal (Sig_{Sum}) erzeugt,
- für mindestens einen Herzschlag, bevorzugt für mehrere Herzschläge, jeweils ein Qualitätsmaß (Q[31]) für die Zuverlässigkeit, mit der der jeweilige charakteristische Herzschlag-Zeitpunkt [H_Zp(x_1), ..., H_Zp(x_N)] eines Herzschlags (x_1, ..., x_N) detektiert worden ist,
- ein Qualitätsmaß (Q[32]) als Maß für die Zuverlässigkeit, mit der ein Referenz-Dämpfungs-Signalabschnitt [Mod(1), ..., Mod(n)] den Beitrag des kardiogenen Signals (Sig_{kar}) zum Zwischen-Signal (Sig_{com}) in einem Herzschlag-Zeitraum oder im Referenz-Herzschlag-Zeitraum (H_Zr_{ref}) rechnerisch kompensiert, und
- ein Qualitätsmaß (Q[33]) für die Form des Zwischen-Signalabschnitts [SigA_{com}(x)] für einen Herzschlag und

wobei die Signalverarbeitungseinheit (5) weiterhin dazu ausgestaltet ist,
bei der ersten Alternative den oder jeden Referenz-Dämpfungs-Signalabschnitt [Mod(1), ..., Mod(n)] unter Verwendung mindestens eines Qualitätsmaßes (Q[30], ..., Q[33]) zu berechnen und
bei der zweiten Alternative für jeden in der Nutzphase detektierten Herzschlag den jeweiligen gedämpften Zwischen-Signalabschnitt [SigA_{com,d}(x)] unter Verwendung

- des ermittelten Referenz-Dämpfungs-Signalabschnitts [Mod(1), ..., Mod(n)] und
- mindestens eines Qualitätsmaßes (Q[30], ..., Q[33]) den angepassten Dämpfungs-Signalabschnitt [Mod(1)(x), ..., Mod(n)(x)] dergestalt zu berechnen, dass
- der angepasste Dämpfungs-Signalabschnitt [Mod(1)(x), ..., Mod(n)(x)] kleiner als oder höchstens genauso groß wie der Referenz-Dämpfungs-Signalabschnitt [Mod(1), ..., Mod(n)] ist und
- umso kleiner ist, je kleiner ein verwendetes Qualitätsmaß (Q[30], ..., Q[33]) ist.

2. Signalverarbeitungseinheit (5) nach Anspruch 1,
**dadurch gekennzeichnet, dass**

die Signalverarbeitungseinheit (5) dazu ausgestaltet ist,

- eine Stichprobe mit mehreren Stichprobenelementen dergestalt zu erzeugen,
dass jedes Stichprobenelement sich auf jeweils einen Herzschlag bezieht und jeweils einen Zwischen-Signalabschnitt [SigA_{com}(x)] als Abschnitt des Zwischen-Signals (Sig_{com}) umfasst, wobei der Abschnitt im Herzschlag-Zeitraum [H_Zr(x)] dieses Herzschlags liegt, und
- für jedes Stichprobenelement jeweils ein Leistungsmaß-Stichprobenelement zu generieren, das ist der zeitliche Verlauf eines Maßes für die elektrische Leistung im Herzschlag-Zeitraum [H_Zr(x)] des Herzschlags,

wobei die Signalverarbeitungseinheit (5) dazu ausgestaltet ist, bei der Berechnung des oder eines Referenz-Dämpfungs-Signalabschnitt [Mod(1), ..., Mod(n)]
einen durchschnittlichen Leistungs-Signalabschnitt [Pow_{com,av}(i)] als ein Mittel über die Leistungsmaß-Stichprobenelemente [zu erzeugen und

den Referenz-Dämpfungs-Signalabschnitt [Mod(1), ..., Mod(n)] unter Verwendung des durchschnittlichen Leistungs-Signalabschnitts [$Pow_{com,av}(i)$] zu berechnen,

insbesondere den durchschnittlichen Leistungs-Signalabschnitt [$Pow_{com,av}(i)$] als den Referenz-Dämpfungs-Signalabschnitt [Mod(1), ..., Mod(n)] zu verwenden, und

zu bewirken, dass der Referenz-Dämpfungs-Signalabschnitt [Mod(1), ..., Mod(n)] im Datenspeicher (9) abgespeichert wird.

3. Signalverarbeitungseinheit (5) nach Anspruch 2,
   **dadurch gekennzeichnet, dass**

   die Signalverarbeitungseinheit (5) weiterhin dazu ausgestaltet ist, bei der ersten Alternative das Mittel über die Leistungsmaß-Stichprobenelemente als ein gewichtetes Mittel zu berechnen,

   wobei die Signalverarbeitungseinheit (5) weiterhin dazu ausgestaltet ist,

   die Gewichtsfaktoren für die Berechnung des durchschnittlichen Leistungs-Signalabschnitts [$Pow_{com,av}(i)$] unter Verwendung jeweils eines Leistungs-Qualitätsmaßes (Q[32]) für jedes Leistungsmaß-Stichprobenelement dergestalt zu berechnen,

   dass der Gewichtsfaktor umso kleiner ist, je kleiner das Leistungs-Qualitätsmaß (Q[32]) ist.

4. Signalverarbeitungseinheit (5) nach Anspruch 3,
   **dadurch gekennzeichnet, dass**

   das Qualitätsmaß (Q[32]), das zur Berechnung der Gewichtsfaktoren verwendet wird,

   das Maß für die Zuverlässigkeit ist, mit der ein Referenz-Dämpfungs-Signalabschnitt [Mod(1), ..., Mod(n)] den Beitrag des kardiogenen Signals ($Sig_{kar}$) zum Zwischen-Signal ($Sig_{com}$) im Referenz-Herzschlag-Zeitraum ($H\_Zr_{ref}$) rechnerisch kompensiert,

   wobei das Maß insbesondere ein Qualitätsmaß für die Form des durchschnittlichen Leistungs-Signalabschnitts [$Pow_{com,av}(i)$] oder eines Leistungsmaß-Stichprobenelements ist.

5. Signalverarbeitungseinheit (5) nach einem der vorhergehenden Ansprüche, **dadurch gekennzeichnet, dass**

   mehrere Frequenzbänder vorgegeben werden und
   die Signalverarbeitungseinheit (5) dazu ausgestaltet ist,
   für jedes vorgegebene Frequenzband jeweils einen Referenz-Dämpfungs-Signalabschnitt-Anteil [Mod(1), ..., Mod(n)] zu berechnen oder durch einen Lesezugriff auf einen Datenspeicher (9) zu ermitteln,

   wobei die Signalverarbeitungseinheit (5) dazu ausgestaltet ist, für jedes vorgegebene Frequenzband und für jeden detektierten Herzschlag, der in die Nutzphase fällt,

   - unter Verwendung des für dieses Frequenzband vorgegebenen oder berechneten Referenz-Dämpfungs-Signalabschnitt-Anteils [Mod(1), ..., Mod(n)] und des oder mindestens eine Qualitätsmaßes (Q[30], ..., Q[33]) jeweils mindestens einen angepassten Dämpfungs-Signalabschnitt-Anteil [Mod(1)(x), ..., Mod(n)(x)] für diesen Herzschlag und für dieses Frequenzband zu berechnen,

   wobei der angepasste Dämpfungs-Signalabschnitt-Anteil [Mod(1)(x), ..., Mod(n)(x)] mit dem durchschnittlichen zeitlichen Verlauf des Beitrags des kardiogenen Signals ($Sig_{kar}$) im Frequenzband zum Zwischen-Signal ($Sig_{com}$) im Herzschlag-Zeitraum ($H\_Zr(x)$) korreliert und

   - jeweils einen in diesem Frequenzband auftretenden Anteil [$SigA_{com}(1)(x)$, ..., $SigA_{com}(n)(x)$] des Zwischen-Signalabschnitts [$SigA_{com}(x)$] für den Herzschlag-Zeitraum [$H\_Zr(x)$] dieses detektierten Herzschlags zu erzeugen und

   - aus dem in diesem Frequenzband auftretenden Anteil [$SigA_{com}(1)(x)$, ..., $SigA_{com}(n)(x)$] des Zwischen-Signalabschnitts [$SigA_{com}(x)$]

      unter Verwendung des angepassten Dämpfungs-Signalabschnitt-Anteils [Mod(1)(x), ..., Mod(n)(x)] für dieses Frequenzband

      einen in diesem Frequenzband auftretenden Anteil [$SigA_{com,d}(1)(x)$, ..., $SigA_{com,d}(n)(x)$] des gedämpften Zwischen-Signalabschnitts [$SigA_{com,d}(x)$] für den Herzschlag-Zeitraum [$H\_Zr(x)$] zu erzeugen.

6. Anordnung umfassend

   - mindestens einen Summen-Signal-Sensor (2.1.1 bis 2.2.2) und

- eine Signalverarbeitungseinheit (5) nach einem der vorhergehenden Ansprüche,

    wobei der oder jeder verwendete Summen-Signal-Sensor (2.1.1 bis 2.2.2) dazu ausgestaltet ist, ein im oder am Körper des Patienten (P) erzeugtes Signal zu messen, und
    wobei die Signalverarbeitungseinheit (5) dazu ausgestaltet ist,

- Messwerte von dem oder jedem Summen-Signal-Sensor (2.1.1 bis 2.2.2) zu empfangen und
- unter Verwendung von empfangenen Messwerten das oder jedes Summen-Signal ($Sig_{Sum}$) zu erzeugen.

7.  Verfahren zum Ermitteln einer Schätzung ($Sig_{res,est}$) für ein respiratorisches Signal ($Sig_{res}$),

    wobei das respiratorische Signal ($Sig_{res}$) mit der Ventilation der Lunge eines Patienten (P) korreliert und die Ventilation der Lunge durch eine eigene Atmungsaktivität und / oder durch eine künstliche Beatmung des Patienten (P) verursacht wird,
    wobei dem Verfahren ein Referenz-Herzschlag-Zeitraum (H_Zr$_{ref}$) und eine Nutzphase vorgegeben werden,
    wobei das Verfahren automatisch unter Verwendung einer Signalverarbeitungseinheit (5) durchgeführt wird und die Schritte umfasst, dass die Signalverarbeitungseinheit (5)
    Messwerte von mindestens einem Summen-Signal-Sensor (2.1.1 bis 2.2.2) empfängt,
    wobei der oder jeder verwendete Summen-Signal-Sensor (2.1.1 bis 2.2.2) ein im und / oder am Körper des Patienten (P) erzeugtes Signal misst,
    unter Verwendung von empfangenen Messwerten ein Summen-Signal ($Sig_{Sum}$) erzeugt,
    wobei das Summen-Signal ($Sig_{Sum}$) eine Überlagerung

        - des zu schätzenden respiratorischen Signals ($Sig_{res}$) und
        - eines kardiogenen Signals ($Sig_{kar}$), welches mit der Herzaktivität des Patienten (P) korreliert,

    umfasst,
    unter Verwendung des Summen-Signals ($Sig_{Sum}$)

        - mehrere Herzschläge und
        - für jeden detektierten Herzschlag jeweils einen charakteristischen Herzschlag-Zeitraum [H_Zr(x)], in dem dieser Herzschlag stattfindet,

    detektiert,
    ein Zwischen-Signal ($Sig_{com}$) berechnet und für die Berechnung des Zwischen-Signals ($Sig_{com}$) den Einfluss der Herzaktivität auf das Summen-Signal ($Sig_{Sum}$) wenigstens näherungsweise rechnerisch kompensiert,
    in einer ersten Alternative mindestens einen Referenz-Dämpfungs-Signalabschnitt [Mod(1), ..., Mod(n)] berechnet und in einer zweiten Alternative den oder jeden Referenz-Dämpfungs-Signalabschnitt [Mod(1), ..., Mod(n)] durch einen Lesezugriff auf einen Datenspeicher (9) ermittelt,
    wobei der oder jeder Referenz-Dämpfungs-Signalabschnitt [Mod(1), ..., Mod(n)] mit dem durchschnittlichen zeitlichen Verlauf des Beitrags des kardiogenen Signals ($Sig_{kar}$) zum Zwischen-Signal ($Sig_{com}$) im Referenz-Herzschlag-Zeitraum (H_Zr$_{ref}$) korreliert,
    für jeden detektierten Herzschlag [H_Zr(x), H_Zr(y)], der in die Nutzphase fällt,

        - jeweils einen Zwischen-Signalabschnitt [SigA$_{com}$(x)] als ein Abschnitt des Zwischen-Signals ($Sig_{com}$) erzeugt,
        wobei der Zwischen-Signalabschnitt [SigA$_{com}$(x)] im Herzschlag-Zeitraum [H_Zr(x), H_Zr(y)] dieses Herzschlags liegt, und
        - aus dem Zwischen-Signalabschnitt [SigA$_{com}$(x)] einen gedämpften Zwischen-Signalabschnitt [SigA$_{com,d}$(x)] für den Herzschlag-Zeitraum [H_Zr(x), H_Zr(y)] erzeugt,
        wobei der Einfluss des kardiogenen Signals ($Sig_{kar}$) auf den gedämpften Zwischen-Signalabschnitt [SigA$_{com,d}$(x)] kleiner als oder höchstens genauso groß wie der Einfluss des kardiogenen Signals ($Sig_{kar}$) auf den Zwischen-Signalabschnitt [SigA$_{com}$(x)] ist, und

    die gedämpften Zwischen-Signalabschnitte [SigA$_{com,d}$(x)] unter Verwendung der detektierten charakteristischen Herzschlag-Zeiträume [H_Zr(x), H_Zr(y)] zur Schätzung ($Sig_{res,est}$) für das respiratorische Signal ($Sig_{res}$) zusammensetzt,
    wobei die Signalverarbeitungseinheit (5) für jeden detektierten Herzschlag

- bei der ersten Alternative den Referenz-Dämpfungs-Signalabschnitt [Mod(1), ..., Mod(n)] und
- bei der zweiten Alternative einen angepassten Dämpfungs-Signalabschnitt [Mod(1)(x), ..., Mod(n)(x)]

auf den jeweiligen Zwischen-Signalabschnitt [SigA$_{com}$(x)] anwendet und durch die Anwendung den gedämpften Zwischen-Signalabschnitt [SigA$_{com,d}$(x)] für den Herzschlag erzeugt,
wobei das Verfahren die weiteren Schritte umfasst, dass die Signalverarbeitungseinheit (5)
mindestens eines der folgenden Qualitätsmaße (Q[30], Q[31], Q[32], Q[33]) berechnet:

- ein Qualitätsmaß (Q[30]) für die Zuverlässigkeit, mit der der oder jeder verwendete Summen-Signal-Sensor (2.1.1 bis 2.2.2) die jeweiligen Messwerte misst und / oder die Zuverlässigkeit, mit der die Signalverarbeitungseinheit (5) aus diesen Messwerten das Summen-Signal (Sig$_{Sum}$) erzeugt,
- für mindestens einen Herzschlag, bevorzugt für mehrere Herzschläge, jeweils ein Qualitätsmaß (Q[31]) für die Zuverlässigkeit, mit der der jeweilige charakteristische Herzschlag-Zeitpunkt [H_Zp(x$_1$), ..., H_Zp(x$_N$)] eines Herzschlags (x$_1$, ..., x$_N$) detektiert worden ist,
- ein Qualitätsmaß (Q[32]) für die Zuverlässigkeit, mit der ein Referenz-Dämpfungs-Signalabschnitt [Mod(1), ..., Mod(n)] den Beitrag des kardiogenen Signals (Sig$_{kar}$) zum Zwischen-Signal (Sig$_{com}$) im Referenz-Herzschlag-Zeitraum (H_Zr$_{ref}$) rechnerisch kompensiert, und
- ein Qualitätsmaß (Q[33]) für die Form des Zwischen-Signalabschnitts [SigA$_{com}$(x)] für einen Herzschlag und

bei der ersten Alternative den oder jeden Referenz-Dämpfungs-Signalabschnitt [Mod(1), ..., Mod(n)] unter Verwendung mindestens eines Qualitätsmaßes (Q[30], ..., Q[33]) berechnet und
bei der zweiten Alternative für jeden in der Nutzphase detektierten Herzschlag den gedämpften Zwischen-Signalabschnitt [SigA$_{com,d}$(x)] unter Verwendung

- des ermittelten Referenz-Dämpfungs-Signalabschnitts [Mod(1), ..., Mod(n)] und
- mindestens eines Qualitätsmaßes (Q[30], ..., Q[33])

den angepassten Dämpfungs-Signalabschnitt [Mod(1)(x), ..., Mod(n)(x)] dergestalt berechnet, dass der angepasste Dämpfungs-Signalabschnitt [Mod(1)(x), ..., Mod(n)(x)]

- kleiner als oder höchstens genauso groß wie der Referenz-Dämpfungs-Signalabschnitt [Mod(1), ..., Mod(n)] ist und
- umso kleiner ist, je kleiner ein verwendetes Qualitätsmaß (Q[30], ..., Q[33]) ist.

8. Verfahren nach Anspruch 7,
**dadurch gekennzeichnet, dass**

das Verfahren die zusätzlichen Schritte umfasst, dass die Signalverarbeitungseinheit (5)

- eine Stichprobe mit mehreren Stichprobenelementen dergestalt erzeugt,

dass jedes Stichprobenelement sich auf jeweils einen Herzschlag bezieht und jeweils einen Zwischen-Signalabschnitt [SigA$_{com}$(x)] als Abschnitt des Zwischen-Signals (Sig$_{com}$) umfasst,
wobei der Abschnitt im Herzschlag-Zeitraum [H_Zr(x)] dieses Herzschlags liegt, und

- für jedes Stichprobenelement jeweils ein Leistungsmaß-Stichprobenelement generiert,
das ist der zeitliche Verlauf eines Maßes für die elektrische Leistung im Herzschlag-Zeitraum [H_Zr(x)] des Herzschlags, und

wobei der Schritt, dass die Signalverarbeitungseinheit (5) den oder einen Referenz-Dämpfungs-Signalabschnitt [Mod(1), ..., Mod(n)] berechnet,
die Schritte umfasst, dass die Signalverarbeitungseinheit (5)

- einen durchschnittlichen Leistungs-Signalabschnitt [Pow$_{com,av}$(i)] als ein Mittel über die Leistungsmaß-Stichprobenelemente erzeugt,
- den Referenz-Dämpfungs-Signalabschnitt [Mod(1), ..., Mod(n)] unter Verwendung des durchschnittlichen Leistungs-Signalabschnitts [Pow$_{com,av}$(i)] berechnet,

insbesondere den durchschnittlichen Leistungs-Signalabschnitt [$Pow_{com,av}(i)$] als den Referenz-Dämpfungs-Signalabschnitt [Mod(1), ..., Mod(n)] verwendet, und

- bewirkt, dass der Referenz-Dämpfungs-Signalabschnitt [Mod(1), ..., Mod(n)] im Datenspeicher (9) abgespeichert wird.

9. Verfahren nach Anspruch 8,
**dadurch gekennzeichnet, dass**

bei der ersten Alternative das Mittel über die Leistungsmaß-Stichprobenelemente ein gewichtetes Mittel ist, wobei die Gewichtsfaktoren für die Berechnung des durchschnittlichen Leistungs-Signalabschnitts [$Pow_{com,av}(i)$] unter Verwendung mindestens eines Qualitätsmaßes (Q[32]) dergestalt berechnet werden, dass der Gewichtsfaktor umso kleiner ist, je kleiner das Qualitätsmaß (Q[32]) ist.

10. Verfahren nach Anspruch 9,
**dadurch gekennzeichnet, dass**

das oder ein Qualitätsmaß (Q[32]), das zur Berechnung der Gewichtsfaktoren verwendet wird, das Maß für die Zuverlässigkeit ist, mit der ein Referenz-Dämpfungs-Signalabschnitt [Mod(1), ..., Mod(n)] den Beitrag des kardiogenen Signals ($Sig_{kar}$) zum Zwischen-Signal ($Sig_{com}$) im Referenz-Herzschlag-Zeitraum ($H\_Zr_{ref}$) rechnerisch kompensiert, wobei insbesondere das Maß ein Qualitätsmaß für die Form des durchschnittlichen Leistungs-Signalabschnitts [$Pow_{com,av}(i)$] ist.

11. Verfahren nach einem der Ansprüche 7 bis 10,
**dadurch gekennzeichnet, dass**

mehrere Frequenzbänder vorgegeben werden,
das Verfahren die zusätzlichen Schritte umfasst, dass
die Signalverarbeitungseinheit (5) für jedes vorgegebene Frequenzband

- bei der ersten Alternative jeweils einen Anteil [Mod(1), ..., Mod(n)] des Referenz-Dämpfungs-Signalabschnitts berechnet und
- bei der zweiten Alternative den Anteil [Mod(1), ..., Mod(n)] durch einen Lesezugriff auf einen Datenspeicher (9) ermittelt,

wobei der Anteil sich auf das Frequenzband bezieht, und
das Verfahren weiterhin die zusätzlichen Schritte umfasst, dass
die Signalverarbeitungseinheit (5) für jedes vorgegebene Frequenzband und für jeden detektierten Herzschlag, der in die Nutzphase fällt,

- jeweils einen in diesem Frequenzband auftretenden Anteil [$SigA_{com}(1)(x)$, ..., $SigA_{com}(n)(x)$] des Zwischen-Signalabschnitts [$SigA_{com}(x)$] für den Herzschlag-Zeitraum [$H\_Zr(x)$] dieses detektierten Herzschlags erzeugt und
- aus dem in diesem Frequenzband auftretenden Anteil [$SigA_{com}(1)(x)$, ..., $SigA_{com}(n)(x)$]

einen in diesem Frequenzband auftretenden Anteil [$SigA_{com,d}(1)(x)$, ..., $SigA_{com,d}(n)(x)$] des gedämpften Zwischen-Signalabschnitts [$SigA_{com,d}(x)$] für den Herzschlag-Zeitraum [$H\_Zr(x)$] erzeugt,
wobei die Signalverarbeitungseinheit (5) für jeden detektierten Herzschlag, der in die Nutzphase fällt, den gedämpften Zwischen-Signalabschnitt [$SigA_{com,d}(x)$] für den Herzschlag-Zeitraum [$H\_Zr(x)$, $H\_Zr(y)$] aus den Anteilen [$SigA_{com,d}(1)(x)$, ..., $SigA_{com,d}(n)(x)$] für die Frequenzbänder zusammensetzt,
wobei die Signalverarbeitungseinheit (5) für jedes vorgegebene Frequenzband
für die Erzeugung des in dem Frequenzband auftretenden Anteils [$SigA_{com,d}(1)(x)$, ..., $SigA_{com,d}(n)(x)$] des gedämpften Zwischen-Signalabschnitts [$SigA_{com,d}(x)$]

- bei der ersten Alternative einen Anteil [Mod(1), ..., Mod(n)] des Referenz-Dämpfungs-Signalabschnitts für das Frequenzband und
- bei der zweiten Alternative einen Anteil [Mod(1)(x), ..., Mod(n)(x)] des angepassten Dämpfungs-Signalabschnitts für das Frequenzband

auf den Anteil [SigA$_{com}$(1)(x), ..., SigA$_{com}$(n)(x)] des Zwischen-Signalabschnitts [SigA$_{com}$(x)] anwendet und wobei die Signalverarbeitungseinheit (5) für jedes vorgegebene Frequenzband

bei der ersten Alternative für jedes vorgegebene Frequenzband den jeweiligen Anteil [Mod(1), ..., Mod(n)] für dieses Frequenzband des Referenz-Dämpfungs-Signalabschnitts unter Verwendung mindestens eines Qualitätsmaßes (Q[30], ..., Q[33]) berechnet und

bei der zweiten Alternative für jeden in der Nutzphase detektierten Herzschlag

den in diesem Frequenzband auftretenden Anteil [SigA$_{com,d}$(1)(x), ..., SigA$_{com,d}$(n)(x)] des gedämpften Zwischen-Signalabschnitts [SigA$_{com,d}$(x)] unter Verwendung des für dieses Frequenzband ermittelten Anteils [Mod(1), ..., Mod(n)] des Referenz-Dämpfungs-Signalabschnitts und des oder mindestens eine Qualitätsmaßes (Q[30], ..., Q[33]) dergestalt berechnet,

dass der Anteil [SigA$_{com,d}$(1)(x), ..., SigA$_{com,d}$(n)(x)] des gedämpften Zwischen-Signalabschnitts [SigA$_{com,d}$(x)]

- kleiner als oder höchstens genauso groß wie der Referenz-Dämpfungs-Signalabschnitt [Mod(1), ..., Mod(n)] für das Frequenzband ist und
- umso kleiner ist, je kleiner ein verwendetes Qualitätsmaß (Q[30], ..., Q[33]) ist.

FIG. 1

FIG. 2

FIG. 3

$Sig_{Sum}$

H_Zp(y)

H_Zp(x)

Atm(1)  Atm(2)  Atm(3)  Atm(4)

300

0

10  20  30  t

FIG. 4

FIG. 5

FIG. 6

EP 4 640 148 A1

FIG. 7

FIG. 8a

FIG. 8b

FIG. 8c

FIG. 8d

FIG. 8e

FIG. 9

FIG. 10

FIG. 11

EP 4 640 148 A1

FIG. 12

FIG. 13

EP 4 640 148 A1

FIG. 14

FIG. 15

FIG. 16

EP 4 640 148 A1

Mod(6)(x)

Mod(6)

EP 4 640 148 A1

FIG. 17

Europäisches
Patentamt

European
Patent Office

Office européen
des brevets

# EUROPÄISCHER RECHERCHENBERICHT

**Nummer der Anmeldung**

EP 25 16 8722

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 10 2022 106326 A1 (DRAEGERWERK AG & CO KGAA [DE]) 6. Oktober 2022 (2022-10-06) * Absätze [0086] - [0095], [0136] - [0138] * ----- | 1-11 | INV. A61B5/08 A61B5/00 |
| A | DE 10 2020 002572 A1 (DRAEGERWERK AG & CO KGAA [DE]) 4. November 2021 (2021-11-04) * Absätze [0098] - [0100], [0124] * * Ansprüche 4, 7 * ----- | 1-11 | |

**RECHERCHIERTE SACHGEBIETE (IPC)**

A61B

Der vorliegende Recherchenbericht wurde für alle Patentansprüche erstellt

| Recherchenort | Abschlußdatum der Recherche | Prüfer |
|---|---|---|
| Den Haag | 11. September 2025 | Schwenke, Stephanie |

KATEGORIE DER GENANNTEN DOKUMENTE

X : von besonderer Bedeutung allein betrachtet
Y : von besonderer Bedeutung in Verbindung mit einer
     anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
     nach dem Anmeldedatum veröffentlicht worden ist
D : in der Anmeldung angeführtes Dokument
L : aus anderen Gründen angeführtes Dokument

.........................................................................................
& : Mitglied der gleichen Patentfamilie, übereinstimmendes
     Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 25 16 8722

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

11-09-2025

| Im Recherchenbericht angeführtes Patentdokument | Datum der Veröffentlichung | Mitglied(er) der Patentfamilie | Datum der Veröffentlichung |
|---|---|---|---|
| DE 102022106326 A1 | 06-10-2022 | DE 102022106326 A1 | 06-10-2022 |
| | | US 2022323017 A1 | 13-10-2022 |
| DE 102020002572 A1 | 04-11-2021 | DE 102020002572 A1 | 04-11-2021 |
| | | US 2021338176 A1 | 04-11-2021 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102009035018 A1
- US 20110028819 A1
- DE 102019006866 A1
- US 20220330837 A1
- DE 102020002572 A1
- US 20210338176 A1